# EUROPEAN PATENT SPECIFICATION

(11) **EP 1 230 338 B1**
(45) Date of publication and mention of the grant of the patent: **08.03.2006**
(21) Application number: 00978814.2
(22) Date of filing: 17.11.2000
(51) Int. Cl.: C12M 1/00, A61K 9/14, A61K 9/50, A61K 9/127, A61K 31/70, A61K 51/00, A61M 36/14, A61F 13/00

(54) **CONTINUOUS-FLOW METHOD FOR PREPARING MICROPARTICLES**
DURCHFLUSSVERFAHREN ZUR HERSTELLUNG VON MIKROPARTIKELN
PROCEDE A DEBIT CONTINU PERMETTANT DE PRODUIRE DES MICROPARTICULES

(30) Priority: 19.11.1999 US 443654
(43) Date of publication of application: 14.08.2002
(73) Proprietor: Zycos Inc., Lexington, MA 02421 (US)
(72) Inventor: HEDLEY, Mary, Lynne, Lexington, MA 02421 (US); HSU, Yung-Yueh, Acton, MA 01720 (US); TYO, Michael, Medford MA 02155 (US)
(74) Representative: Dörries, Hans Ulrich
(86) International application number: PCT/US2000/031770
(87) International publication number: WO 2001/036583

(56) References cited:
- WO-A-98/31398
- GB-A- 2 310 801
- US-A- 5 643 605
- US-A- 6 020 004
- US-A- 6 048 551

## Description

### BACKGROUND OF THE INVENTION

This invention relates to a method for manufacturing polymeric microparticles.

Biodegradable synthetic polymers have been used as drug carriers. It has been observed that, when used as drug carriers, synthetic polymers are generally superior to natural polymers with respect to reproducibility of product release (Okada et al., *Critical Reviews in Therapeutic Drug Carrier Systems* 12(1): 1, 1995).

Several methods have been developed for preparing microspheres of hydrophobic synthetic polymers containing hydrophilic drugs. These methods include (1) emulsion solvent evaporation (e.g., oil/water, water/oil, water/oil/water emulsion evaporation). (2) phase separation, (3) interfacial polymerization, and (4) spray drying (*ibid*.). Patent application WO 98/31398 describes nucleic acid containing microparticles and methods of preparation.

### SUMMARY OF THE INVENTION

The invention is based on the discovery of a method for scalable, continuous flow production of a nucleic acid-containing microparticle that maintains the structural integrity of the associated nucleic acid and results in a microparticle having a purity suitable for introduction into an animal (e.g., human) host. Microparticles prepared according to the continuous flow processes described herein can be used for delivery of a nucleic acid for gene therapy, antisense therapy, vaccination, treatment of autoimmune disease, and either specific or non-specific modulation of an immune response (e.g., via cytokine regulation). The microparticles can additionally be used to deliver nucleic acid encoding a protein or peptide useful in any type of therapy.

Accordingly, in one aspect the invention features a scalable continuous process for preparing nucleic acid-containing microparticles. The process includes the steps of: (a) providing a mixing chamber and a solvent removal device: (b) continuously supplying a first emulsion to the mixing chamber (i.e., where the first emulsion includes (i) an organic solution, including a polymeric material and an organic solvent (e.g.. dichloromethane, "DCM"), mixed with (ii) a first aqueous solution, including a nucleic acid); (c) continuously supplying a second aqueous solution, including a surfactant, to the mixing chamber; (d) continuously emulsifying the first emulsion and the second aqueous solution in the mixing chamber to form a second emulsion, which includes nucleic acid polymeric material water, and organic solvent; (e) continuously transferring the second emulsion from the mixing chamber to the solvent removal device; and (f) removing the organic solvent from the second emulsion in the solvent removal device to form an aqueous suspension of nucleic acid-containing microparticles. The first emulsion, the second aqueous solution, or both further includes a stabilizer.

Alternatively, step (b) can be carried out where the nucleic acid is not in aqueous solution (e.g., where the nucleic acid is dry). An advantage of carrying out this step with dry nucleic acid is that doing so can lead to a high encapsulation efficiency (e.g., near 100%).

In some embodiments, steps (b) and/or (d) are carried out at a temperature between about 2°C and about 8°C.

The average residence time of the first emulsion and the second aqueous solution in the mixing chamber can be less than about 60 seconds (e.g., less than 60 seconds, less than 45 seconds, less than 30 seconds, less than 20 seconds, less than 15 seconds, less than 10 seconds, less than 5 seconds, less than 2 seconds, or less than 1 second). The average residence time of the second emulsion in the solvent removal device can be less than about 3 hours (e.g., less than 3 hours, less than 2 hours, less than 1 hour, less than 30 minutes, or less than 15 minutes). For the purposes of this application, "average residence time" is defined as the flow rate to volume ratio.

The first aqueous solution and the second aqueous solution can be, for example, of essentially equal osmolarity, buffering capacity, and pH. The second aqueous solution can, optionally, include polyvinyl alcohol (PVA) and/or a carbohydrate such as sucrose. For the purposes of this application, the term "essentially equal" denotes less than about a 10% difference of one parameter of the first solution from the same parameter of the second solution. Thus, "essentially equal osmolarity" means that the number of particles per unit volume are within about 10% of each other, while "essentially equal pH" means pH units within about 10% other (e.g., pH 7.5 and 8.0 would be essentially equal pH) and "essentially equal buffering capacity" means less than about 10% difference in concentration of buffer components. For the purposes of this application, "essentially equal" and "essentially identical" are interchangeable terms.

The polymeric material can be, for example, a synthetic, biodegradable polymer such as poly-lactic-*co*-glycolic acid, "PLGA". The ratio of lactic acid to glycolic acid in the PLGA can be, for example, between about 1:2 and about 4:1 by weight (e.g., about 1:1), and the PLGA an have an average molecular weight in the range of, for example, about 6,000 to 100.000.

The stabilizer can be a single compound or a mixture of compounds (e.g., a carbohydrate such as sucrose and a buffer such as TRIS-EDTA, "TE"). The stabilizer can alternatively or additionally include a lipid. The lipid can be, e.g., a cationic lipid, an anionic lipid, or a zwitterionic lipid, or may have no charge. Examples of lipids include cetyltrimethylammonium bromide (CTAB) and phospholipids, e.g., phosphatidylcholine. Specific examples include polyethylene glycol distearoylphosphatidyl ethanolamine (PEG-DSPE), 3-[(3-cholamidopropyl)-dimethylammonio]-1-propane-sulfonate (CHAPS), taurocholic acid, glycocholic acid, capric acid. N-lauryl sarcosine, fatty acyl carnitine and vitamin D3. The microparticles may contain one or more than one type of lipid, e.g., those lipids present in lecithin lipid preparations and may also include one or more additional stabilizers.

The lyphilized or freeze-dried microparticles can, for example, have a residual organic solvent level of less than about 200 ppm (e.g., less than 200 ppm, less than 150 ppm, less than 100 ppm, less than 50 ppm, less than 20 ppm, or less than 10 ppm). Alternatively; microparticles can be air-dried and can have about the same residual organic solvent levels.

In some embodiments, the process described above also includes the steps of: (g) providing a diafiltration apparatus (e.g., including a hollow fiber system); (h) diluting the aqueous suspension with an aqueous wash solution; (i) supplying the diluted aqueous suspension to the diafiltration apparatus; and (j) removing an aqueous waste solution (i.e., including at least some of the wash solution of step (h)) from the diluted aqueous suspension in the diafiltration apparatus, to form a purified aqueous suspension that includes nucleic acid-containing microparticles. The process can further include some or all the steps of: washing the purified aqueous suspension to form a suspension of washed microparticles; concentrating the suspension of washed microparticles or the purified aqueous suspension in the diafiltration apparatus, to form a concentrate; and transferring the concentrate into one or more vessels. The process can still further include the step of: (m) lyophilizing, freeze-drying, or air drying the concentrate aqueous suspension in the one or more vessels, to form lyophilized, freeze-dried, or air-dried microparticles. Steps (h), (i) and (j) can optionally be carried out at a temperature of between about 2°C and about 8°C.

In other embodiments, the process described above also includes the steps of: (g) contacting the aqueous suspension with a vibrating or non-vibrating fine-mesh screen (e.g.. as in a fine mesh stainless steel screen contained within a Sweco device or a cartridge filter); (h) filtering the aqueous suspension through the screen to remove at least some of each of the first and second aqueous solutions and to retain the microparticles on the screen; (i) washing the microparticles with at least one aqueous wash solution (e.g., water-for-injection, "WFI") to produce washed microparticles; and (j) drying the washed microparticles to produce dried microparticles. The aqueous wash solution can optionally be at a temperature of, for example, about 2°C to about 8°C. The process can also include the step of contacting the washed microparticles with an excipient (e.g., prior to the drying step), and/or the step of transferring the dried microparticles into one or more vessels.

In the above processes, the mixing chamber can, for example, include a homogenizer. The solvent removal device can be a bioreactor. The second aqueous solution can be supplied to the mixing chamber at a flow rate of between 0.1 and 100 l/min (e.g., 0.1 to 20 l/min or 0.1 to 50 l/min).

The organic solvent can be removed from the second emulsion in the solvent removal device, for example, by evaporation, by heating the second emulsion in the solvent removal device (e.g., to between 30°C and 55°C), by an extraction process, by applying a partial vacuum to the solvent removal device, or any combination of these methods. Alternatively or additionally, the removal of the organic solvent from the second emulsion in the solvent removal device can be facilitated by diluting the second emulsion in the solvent removal device.

For the purposes of this application, a "solvent removal device" is a device that accomplishes removal of the solvent from microparticles, but not necessarily from the fluid in which they are suspended. Examples of suitable solvent removal devices include a bioreactor, a tank (e.g. a hardening tank), a hollow fiber cartridge and a device that contain the microparticles and have water passed through it (e.g. a Sweco device).

In any of the processes described above, each of the steps can be carried out aseptically.

In any of the processes described above, at least about 50% (e.g.. 50%. 60%. 70%. 80%. 90%, 95%, 99%, or more) of the nucleic acid in the microparticles can be in the form of circular RNA molecules or supercoiled circular DNA molecules.

The average diameter of microparticles can be, for example, less than about 100 microns (e.g., less than 100 microns, less than 50 microns, less than 20 microns, between about 0.5 and about 2.5 microns, inclusive, or smaller), whether measured according to number average or volume average.

*Biodegradable* is used here to mean that the polymers degrade over time into non-toxic compounds that are cleared from the host cells by normal metabolic pathways. Generally, a biodegradable polymer will be substantially metabolized within one to three months after injection into a patient, and essentially completely metabolized within about two years.

*Essentially identical* in the context of a DNA or polypeptide sequence is defined here to mean differing no more than 25% from the naturally occurring sequence, when the closest possible alignment is made with the reference sequence and where the differences do not adversely affect the desired function of the DNA or polypeptide in the methods of the invention. The phrase *fragment* o*f a protein* is used to denote some portion of the protein that is at least four residues in length, but less than the whole protein.

The determination of percent homology between two sequences is accomplished using the algorithm of Karlin et al., *Proc. Natl. Acad Sci. USA* 87:2264-2268, 1990, modified as in Karlin et al., *Proc. Natl. Acad Sci. USA* 90:5873-5877, 1993. Such an algorithm is incorporated into the NBLAST and XBLAST programs of Altschul et al., *J*. *Mol. Biol.* 215:403-410, 1990, BLAST nucleotide searches are performed with the NBLAST program, score = 100, wordlength = 12, to obtain nucleotide sequences homologous to a nucleic acid molecule of the invention. BLAST protein searches are performed with the XBLAST program, score = 50, wordlength = 3. to obtain amino acid sequences homologous to a protein molecule of the invention. To obtain gapped alignments for comparison purposes, Gapped BLAST is utilized as described in Altschul et al., *Nucleic Acids Res.* 25:3389-3402, 1997. When utilizing BLAST and Gapped BLAST programs, the default parameters of the respective programs (e.g., XBLAST and NBLAST) should be used. *See* http://www.ncbi.nlm.nih.gov.

The peptide or polypeptide encoded by the nucleic acid can be linked to a targeting sequence. The term *targeting sequence* is used interchangeably with trafficking sequence and refers to an amino acid sequence that causes a polypeptide to which it is fused to be transported to a specific compartment of the cell, e.g., the nucleus, the endoplasmic reticulum the golgi apparatus, an intracellular vesicle, a lysosome, or an endosome.

In the embodiment where the expression product includes a peptide having a length and sequence which permit it to bind an MHC class I or II molecule, the expression product is typically immunogenic or immunosuppressive. The expression product can have an amino acid sequence that differs in sequence identity by up to 25% from the sequence of a naturally occurring peptide or protein recognized by a T cell, provided that it can still be recognized by the same T cell. Such variant peptides may function exactly as the naturally occurring counterpart, or instead may act by altering the cytokine profile of the T cell (i.e., an "altered peptide ligand"). The differences between the expression product and the naturally occurring peptide can, for example, be engineered to increase cross-reactivity to pathogenic viral strains, to remove or alter amino acids that give the protein an undesirable function, or to increase HLA-allotype binding.

Examples of *expression products* include amino acid sequences at least 50% identical to the sequence of an MHC class II binding fragment of myelin basic protein (MBP), proteolipid protein (PLP), invariant chain, GAD65, islet cell antigen, desmoglein, α-crystallin, or β-crystallin. Table 1 lists many proteins that are thought to be involved in autoimmune disease. For example, the fragments may be essentially identical to any one of SEQ ID NOS: 1-46, such as MBP residues 80-102 (SEQ ID NO: 1), PLP residues 170-191 (SEQ ID NO: 2), or invariant chain residues 80-124 (SEQ ID NO: 3). Other examples of fragments are listed in Table 2.

Alternatively, the expression product can include an amino acid sequence essentially identical to the sequence of an antigenic portion of any of the tumor antigens listed in Table 3. such as those encoded by the human papilloma virus E1, E2, E6, and E7 genes. Her2/neu gene, the prostate specific antigen gene, the melanoma antigen recognized by T cells (MART) gene, or the melanoma antigen gene (MAGE). Again, the expression product can be engineered to increase cross-reactivity.

**TABLE 1: Autoantigens**

| **Disease** | | **Associated Antigen** | **Notes** |
|---|---|---|---|
| Coeliac disease | | α-Gliadin | a |
| Goodpasture's syndrome | | Basement membrane collagen | a |
| Graves' disease | | Thyroid Stimulating Hormone (TSH) receptor | a |
| Hashimoto's disease | | Thyroglobulin | a |
| Isaac's syndrome | | voltage-gated potassium channels | b |
| Insulin-dependent | | Glutamic acid decarboxylase (GAD) | a |
| | diabetes | Insulin receptor | a |
| | | Insulin associated antigen (IA-w) | a |
| | | Hsp | b |
| Lambert-Eaton myasthenic | | Synaptogamin in voltage-gated calcium | |
| | syndrome (LEMS) | channels | b |
| Multiple sclerosis | | Myelin basic protein (MBP) | a |
| | | Proteolipid protein (PLP) | a |
| | | Myelin oligodendrocyte-associated | |
| | | protein (MOG) | a |
| | | αB-crystallin | a |
| Myasthenia gravis | | Acetyl choline receptor | a |
| Paraneoplastic | | RNA-binding protein HuD | b |
| | encephalitis | | |
| Pemphigus vulgaris | | "PeV antigen complex" | a |
| | | Desmoglein (DG) | c |
| Primary biliary cirrhosis | | Dihydrolipoamide acetyltransferase | b |
| | | Pyruvate dehydrogenase complex 2 (PDC-E2) | d |
| Progressive systemic | | DNA topoisomerase | a |
| | sclerosis | RNA polymerase | a |
| Rheumatoid arthritis | | lmmunoglobulin Fc | a |
| | | Collagen | |
| Scleroderma | | Topoisomerase I | b |
| Stiff-man syndrome | | Glutamic acid decarboxylase (GAD) | a |
| Systemic lupus | | ds-DNA | a |
| | erythematosus | | |
| Uveitis | | lnterphotoreceptor retinoid-binding protein | b |
| | | S antigen (rod out segment) | b |

| | | | |
|---|---|---|---|
| References: | | | |
| a) HLA and Autoimmune Disease. R. Heard, pg. 123-151 in HLA & Disease. Academic Press. New York, 1994, (R. Lechler, ed.) | | | |
| b) *Cell* 80:7-10(1995) | | | |
| c) *Cell* 67:869-877 (1991) | | | |
| d) *JEM* 181: 1835-1845 (1995) | | | |

**TABLE 2: Class II Associated Peptides**

| **Peptide** | **SEQ ID NO:** | **Source Protein** |
|---|---|---|
| GRTQDENPVVHFFKNIVTPRTPP | I | MBP 80-102 |
| AVYVYIYFNTWTTCQFIAFPFK | 2 | PLP 170-191 |
| FKMRMATPLLMQA | 3 | Invariant chain 88-100 |
| TVGLQLIQLINVDEVNQIV | | |
| TTNVRLKQQWVDYNLKW | 4 | Achr α 32-67 |
| QIVTTNVRLKQQWVDYNLKW | 5 | Achr α 48-67 |
| QWVDYNL | 6 | Achr α 59-65 |
| GGVKKIHIPSEKIWRPDL | 7 | Achr α 73-90 |
| AIVKFTKVLLQY | 8 | Achr α 101-112 |
| WTPPAIFKSYCEIIVTHFPF | 9 | Achr α 118-137 |
| MKLGTWTYDGSVV | 10 | Achr α 144-156 |
| MKLGIWTYDGSVV | 11 | Achra 144-157 analog(1-148) |
| WTYDGSVVA | 12 | Achr α 149-157 |
| SCCPDTPYLDITYHFVM | 13 | Achr α 191-207 |
| DTPYLDITYHFVMQRLPL | 14 | Achr α 195-212 |
| FIVNVIIPCLLFSFLTGLVFY | 15 | Achr α 214-234 |
| LLVIVELIPSTSS | 16 | Achr α 257-269 |
| STHVMPNWVRKVFIDTIPN | 17 | Achr α 304-322 |
| NWVRKVFIDTIPNIMFFS | 18 | Achr α 310-327 |
| IPNIMFFSTMKRPSREKQ | 19 | Achr α 320-337 |
| AAAEWKYVAMVMDHIL | 20 | Achr α 395-410 |
| IIGTLAVFAGRLIELNQQG | 21 | Achr α 419-437 |
| GQTIEWIFIDPEAFTENGEW | 22 | Achr γ 165-184 |
| MAHYNRVPALPFPGDPRPYL | 23 | Achr γ 476-495 |
| LNSKIAFKIVSQEPA | 24 | desmoglein 3 190-204 |
| TPMFLLSRNTGEVRT | 25 | desmoglein 3 206-220 |
| PLGFFPDHQLDPAFGA | 26 | HBS preS1 10-25 |
| LGFFPDHQLDPAFGANS | 27 | HBS preS1 11-27 |
| FFLLTRILTI | 28 | HBS Ag 19-28 |
| RILTIPQSLD | 29 | HBS Ag 24-33 |
| TPTLVEVSRNLGK | 30 | HSA 444-456 |
| AKTIAYDEEARR | 31 | hsp 65 2-13 |
| VVTVRAERPG | 32 | hsp 18 61-70 |
| SQRHGSKYLATASTMDHARHG | 33 | MBP 7-27 |
| RDTGILDSIGRFFGGDRGAP | 34 | MBP 33-52 |
| QKSHGRTQDENPVVHFFKNI | 35 | MBP 74-93 |
| DENPVVHFFKNIVT | 36 | MBP 84-97 |
| ENPVVHFFKNIVTPR | 37 | MBP 85-99 |
| HFFKNIVTPRTPP | 38 | MBP 90-102 |
| KGFKGVDAQGTLSK | 39 | MBP 139-152 |
| VDAQGTLSKIFKLGGRDSRS | 40 | MBP 144-163 |
| LMQYIDANSKFIGITELKK | 41 | Tetanus Toxoid 828-846 |
| QYIKANSKFIGIT | 42 | Tetanus Toxoid 830-842 |
| FNNFTVSFWLRVPK | 43 | Tetanus Toxoid 947-960 |
| SFWLRVPKVSASHLE | 44 | Tetanus Toxoid 953-967 |
| KFIIKRYTPNNEIDSF | 45 | Tetanus Toxoid 1174-1189 |
| GQIGNDPNRDIL | 46 | Tetanus Toxoid 1273-1284 |

**TABLE 3: Tumor Antigens**

| **Cancer** | **Associated Antigen** |
|---|---|
| Melanoma | BAGE 2-10 |
| Breast/Ovarian | c-ERB2 (Her2/neu) |
| Burkitt's lymphoma/Hodgkin's lymphoma | EBNA-1 |
| Burkitt's lymphoma/Hodgkin's lymphoma | EBNA-2 |
| Burkitt's lymphoma/Hodgkin's lymphoma | EBNA-3 |
| Burkitt's lymphoma/Hodgkin's lymphoma | EBNA-3A |
| Burkitt's lymphoma/Hodgkin's lymphoma | EBNA-3C |
| Burkitt's lymphoma/Hodgkin's lymphoma | EBNA-4 |
| Burkitt's lymphoma/Hodgkin's lymphoma | EBNA-6 |
| Burkitt's lymphoma/Hodgkin's lymphoma | EBV |
| Burkitt's lymphoma/Hodgkin's lymphoma | EBV LMP2A |
| Melanoma | GAGE- I |
| Melanoma/Renal Cell Carcinoma/Glioma | gp75/TRP1 |
| Cervical | HPV 16 E6 |
| Cervical | HPV 16 E7 |
| Cervical | HPV 18 E6 |
| Cervical | HPV 18 E7 |
| Melanoma | MAG |
| Melanoma | MAGE-1 |
| Melanoma | MAGE-2 |
| Melanoma | MAGE-3 |
| Melanoma | MAGE-4b |
| Melanoma | MAGE-5 |
| Melanoma | MAGE-6 |
| Melanoma | MART-1/Melan-A |
| Pancreatic/Breast/Ovarian | MUC-I |
| Melanoma | MUM-I-B |
| Breast/Colorectal/Burkitt's lymphoma | p53 |
| Melanoma | Pmel 17(gp 100) |
| Prostate | PSA Prostate Specific Antigen |
| Melanoma/Breast/Glioma | Tyrosinase |
| Colorectal/Gastric/Pancreatic/Breast/Lung CEA | Carcinoembryonic Antigen |
| Lung | LRP Lung Resistance Protein |
| Lung/Colorectal/Prostate | hCG |
| Multiple | IGFR-1 |
| Melanoma/Head/Neck/Renal Cell Carcinoma | NY-ESO-I |
| Lung/Ovarian/Bladder/Prostate | MAGE-A3 |
| >85% of Human Cancers | hTERT |
| Breast/Glioma/Gastric/Ovarian/Squamous Cell | EGFR |
| Colorectal/Gastric/Ovarian/Osteosarcoma | CD55 |
| Breast/Ovarian/Prostate/Pancreatic/Bladder | Her-2 |
| Prostate | PAP |

**TABLE 4: Class I associated tumor and pathogen-peptides**

| **Peptide** | **SEQ ID NO:** | **Source Protein** |
|---|---|---|
| AARAVFLAL | 47 | BAGE 2-10 |
| YRPRPRRY | 48 | GAGE-1 9-16 |
| EADPTGHSY | 49 | MAGE-1 161-169 |
| SAYGEPRKL | 50 | MAGE-1 230-238 |
| EVDPIGHLY | 51 | MAGE-3161-169 |
| FLWGPRALV | 52 | MAGE-3 271-279 |
| GIGILTV | 53 | MART-1 29-35 |
| ILTVILGV | 54 | MART-1 32-39 |
| STAPPAHGV | 55 | MUC-1 9-17 |
| EEKLIVVLF | 56 | MUM-1 261-269 |
| MLLAVLYCL | 57 | TYROSINASE 1-9 |
| SEIWRDIDF | 58 | TYROSINASE 192-200 |
| AFLPWHRLF | 59 | TYROSINASE 206-214 |
| YMNGTMSQV | 60 | TYROSINASE 369-376 |
| KTWGQYWQV | 61 | PMEL 17 (GP100) 154-162 |
| ITDQVPFSV | 62 | PMEL 17 (GP100) 209-217 |
| YLEPGPTVA | 63 | PMEL 17(GP100) 280-288 |
| LLDGTATLRL | 64 | PMEL 17 (GP100) 476-48S |
| ELNEALELEK | 65 | p53 343-351 |
| STPPPGTRV | 66 | p53 149-157 |
| LLPENNVLSPL | 67 | p53 25-35 |
| LLGRNSFEV | 68 | p53 264-272 |
| RMPEAAPPV | 69 | p53 65-73 |
| KIFGSLAFL | 70 | HER-2/neu 369-377 |
| IISAVVGIL | 71 | HER-2/neu 654-662 |
| CLTSTVQLV | 72 | HER-2/neu 789-797 |
| YLEDVRLV | 73 | HER-2/neu 835-842 |
| VLVKSPNHV | 74 | HER-2/neu 851-859 |
| RFRELVSEFSRM | 75 | HER-2/neu 968-979 |
| LLRLSEPAEL | 76 | PSA 119-128 |
| DLPTQEPAL | 77 | PSA 136-144 |
| KLQCVD | 78 | PSA 166-171 |
| VLVASRGRAV | 79 | PSA 36-45 |
| VLVHPQWVL | 80 | PSA 49-57 |
| DMSLLKNRFL | 81 | PSA 98-107 |
| QWNSTAFHQ | 82 | HBV envelope 121-130 |
| VLQAGFF | 83 | HBV envelope 177-184 |
| LLLCLIFL | 84 | HBV envelope 250-257 |
| LLDYQGML | 85 | HBV envelope 260-267 |
| LLVPFV | 86 | HBV envelope 338-343 |
| SILSPFMPLL | 87 | HBV envelope 370-379 |
| PLLPIFFCL | 88 | HBV envelope 377-385 |
| ILSTLPETTV | 89 | HBV core 529-538 |
| FLPSDFFPSV | 90 | HBV core 47-56 |
| KLHLYSHPI | 91 | HBV polymerase 489-498 |
| ALMPLYACI | 92 | HBV polymerase 642-651 |
| HLYSHPIIL | 93 | HBV polymerase 1076-1084 |
| FLLSLGIHL | 94 | HBV polymerase 1147-1153 |
| HLLVGSSGL | 95 | HBV polymerase 43-51 |
| GLSRYVARL | 96 | HBV polymerase 455-463 |
| LLAQFTSAI | 97 | HBV polymerase 527-535 |
| YMDDVVLGA | 98 | HBV polymerase 551-559 |
| GLYSSTVPV | 99 | HBV polymerase 61-69 |

| | | |
|---|---|---|
| NLSWL | 100 | HBV polymerase 996-1000 |
| KLPQLCTEL | 101 | HPV 16 E6 18-26 |
| LQTTIHDII | 102 | HPV 16 E6 26-34 |
| FAFRDLCIV | 103 | HPV 16 E6 52-60 |
| YMLDLQPET | 104 | HPV 16 E7 11-19 |
| TLHEYMLDL | 105 | HPV 16 E7 7-15 |
| LLMGTLGIV | 106 | HPV 16 E7 82-90 |
| TLGIVCPI | 107 | HPV 16 E7 86-93 |
| LLMGTLGIVCPI | 108 | HPV 16 E7 82-93 |
| LLMGTLGIVCPICSQK | 109 | HPV 16 E7 82-97 |

In still other cases, the expression product includes an amino acid sequence essentially identical to the sequence of an antigenic fragment of a protein naturally expressed by a virus, e.g., a virus which chronically infects cells, such as human papilloma virus (HPV), human immunodeficiency virus (HIV), herpes simplex virus (HSV), hepatitis B virus (HBV), or hepatitis C virus (HCV); a bacterium, such as mycobacteria or *Helicobacter pylori*; a fungus such as *Candida, Aspergillus. Cryptococcus,* or *Histoplasmosis* species, or other eukaryotes, such as a *Plasmodium* species. A representative list of such class I-binding fragments as well as fragments of tumor antigens is included in Table 4. The MHC binding fragments (class I or class II) can be encoded as part of a larger polytope compound of the type described in USSN 09/398,534 and USSN 60/154,665.

The nucleic acid in the microparticles described herein can be distributed either throughout the microparticle, or in a small number of discrete regions within the microparticle. Alternatively, the nucleic acid can be in the core of a hollow-core microparticle. The microparticle preferably does not contain a cell (e.g., a bacterial cell), or a naturally occurring genome of a cell.

The microparticles can also include a stabilizer. A stabilizer is a compound or compounds that act to protect the nucleic acid (e.g., to keep it supercoiled or protect it from degradation) at some point during the production and/or storage of the microparticles. Examples of stabilizers include carbohydrates such as dextrose, sucrose, and trehalose; polyvinyl alcohol; cyclodextrin; dextran; dextran sulfate; cationic compounds such as cationic peptides; buffering agents such as TRIS, PBS, and MOPS; chelating agents such as EDTA and EGTA; DNase inhibitors; pluronics, e.g., Pluronic F-68 (Sigma-Aldrich Co., St. Louis, MO): and lipids such as CHAPS. PEG-DSPE, taurocholic acid, glycocholic acid, fatty acyl camitine, N- lauryl sarcosine, capric acid, vitamin D3, hexadecyltrimethylammonium bromide, QS21, purified saponin, or polymyxin B.

Stabilizers can also be release modifiers such as carbohydrates, cationic compounds, pluronics, lipids (e.g., membrane destabilizing lipids), proteins, salts, peptides, surfactants, and small molecules. The stabilizer can remain associated with the DNA after the latter is released from the polymeric matrix. Moreover, although the term "stabilizer" is used in the singular form, it should be understood to refer also to mixtures of two or more compounds (e.g., a carbohydrate and a buffer, or a carbohydrate, a buffer, and a lipid).

Among the advantages of the invention is that it provides an economical, aseptic, scalable procedure for producing a microparticle in amounts necessary for research, clinical, and other commercial uses. A microparticle produced using these procedures contains stable, active, potent, structurally intact nucleic acid, e.g., as supercoiled DNA. The method also provides for efficient encapsulation of the nucleic acid in the microparticle and allows for efficient recovery of the microparticle.

Another advantage of the invention is that it affords microparticles that are substantially free of impurities, such as organic solvents used to prepare the microparticle. The microparticles are suitable, therefore, for oral, rectal, vaginal, intranasal, intraarterial, intravenous, pulmonary, intramuscular, intradermal, transmucosal, intrathecal, intraperitoneal, transdermal, subdermal, or subcutaneous delivery.

Still another advantage of the invention is the control afforded over microparticle size, concurrent with scalable and reproducible large-scale production of microparticles. Microparticle size can be important for a number of reasons, including delivery to a particular target site (e.g., lungs via inhalation), intravenous uptake by phagocytotic cells, and stability of the pharmaceutical suspension during storage and treatment. Moreover, the microparticles of the invention can be lyophilized, dried using a Sweco device (Emerson Electric Co., Florence. KY). or stored as a frozen liquid. Drying of microparticles can be accomplished as described in U.S. 6,080,429. Microparticles prepared in this manner are readily resuspended in a wide range of dispersing agents.

The microparticles can be administered to an animal (e.g., a mammal such as a human, non-human primate, horse, cow, pig, sheep, goat, dog, cat mouse, rat, guinea pig, rabbit, hamster, or ferret). The microparticles can be provided suspended in an aqueous solution or any other suitable formulation, and can be, for example, delivered orally, vaginally, rectally, nasally, buccally, or by inhalation, or injected or implanted (e.g., surgically) into the animal. They can optionally be delivered in conjunction with a protein such as a cytokine or interferon, an antigen, a lipid, an adjuvant, or excipients that may be within or without the microparticle.

Other features and advantages of the invention will be apparent from the following detailed description, and from the claims.

### BRIEF DESCRIPTION OF THE DRAWINGS

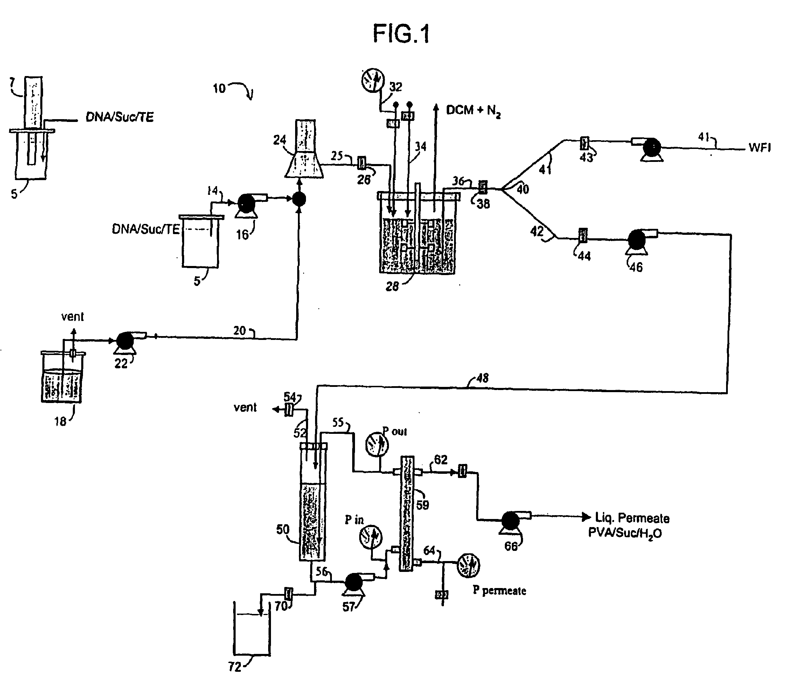

Fig. 1 is a schematic representation of a system for preparing nucleic acid-containing microparticles according to the invention.

### DESCRIPTION OF THE PREFERRED EMBODIMENTS

The microparticles of the invention are formulated so as to maintain the integrity of the nucleic acid. For plasmid DNA, this means maximizing the percentage of plasmid molecules that are supercoiled and thus more stable than non-supercoiled (i.e., nicked or linear) plasmids (Middaugh et al., *J. Pharm. Sci.* 87:130, 1998).

The nucleic acid can be RNA or DNA, or any known derivative of DNA (e.g., phosphorothiolate derivatives and the like typically used in antisense applications). In some embodiments, at least 50% (and preferably at least 70% or even 80%) of the nucleic acid is in the form of closed circles. The nucleic acid can be a linear or circular molecule, and can thus be, e.g., a plasmid, or may include a viral genome, or part of a viral genome. When circular and double-stranded, it can be nicked, i.e., in an open circle, or super-coiled. In some embodiments the nucleic acids are plasmid molecules, at least 25% (and preferably at least 35%, 40%, 50%, 60%, 70%, 80%, or even 90% or more) of which are supercoiled.

The invention is not restricted to the manufacture of a microparticle having a particular type of nucleic acid. Any type of nucleic acid may be used. Examples include DNA which serves as the template for expression of an antisense RNA or a ribozyme, nucleic acids which encode a useful protein or peptide, and nucleic acids which themselves produce the desired effect, such as antisense DNA or RNA, poly I:C, BCG DNA. oligonucleotides, and DNA having immunomodulating potential (e.g., CpG motifs).

The nucleic acid may encode a peptide or polypeptide that modulates, e.g.. elicits, enhances, alters, or suppresses, a humoral or cell-mediated immune response. When modulation of cell-mediated immune responses is desired, the encoded product may be or include, for example, an MHC class 1- or MHC class II-binding peptide or polypeptide. Examples of nucleic acids encoding proteins or peptides that elicit immune responses are described in WO 95/23738: WO 97/17063; U.S. Patent No. 5,880,103; Jones et al., *Vaccine* 15:814, 1997; Chen et al., *J*. *Virology* 72:5757, 1998; Mathowitz et al., *Nature* 386:41, 1997: Jong et al.. *J. Controlled Release* 47:123. 1997; and Hedley et al.. *Nature Med.* 4:365, 1998. USSN 09/398.534. and USSN 60/154,665.

The nucleic acid may alternatively elicit a non-specific immune response. One example of such a nucleic acid is poly I:C (i.e., a polyinosine:polycytosine double stranded nucleic acid), which induces an interferon response (see, e.g., EP A-0248 531). Another example is immunostimulatory CpG-oligodeoxynucleotides, which act as adjuvants for Thl responses and cytotoxic T cell responses to proteinaceous antigens (Sparwasser et al., *Eur.J. Immunol.* 28:2045, 1998). Another example is provided by nucleic acid encoding a polypeptide that regulates immune cells (e.g. T cells).

The nucleic acids can also include oligonucleotides which modulate gene expression. e.g., the antisense oligonucleotides described in Lewis et al...*J*. *Controlled Release* 37:173, 1995.

Alcohol precipitation of the nucleic acid prior to dissolution in the aqueous solution can improve encapsulation efficiency. Precipitation with ethanol can result in up to a 147% increase in incorporated DNA, and precipitation with isopropanol up to 170% (see. e.g., PCT US98/01499).

The nature of the aqueous solution can affect the yield of supercoiled or encapsulated DNA. For example, addition of buffer solutions containing either tris(hydrovymethyl)-aminomethane (TRIS), ethylenediamine-tetraacetic acid (EDTA), or a combination of TRIS and EDTA (TE), results in stabilization of supercoiled plasmid DNA, according to analysis by gel electrophoresis. Moreover, relatively high pH (e.g., 8.0 to 9.9) also has a stabilizing effect. Certain compounds, such as dextran sulfate, dextrose, dextran. CTAB, polyvinyl alcohol, trehalose, lipids, cyclodextrin, and sucrose, can enhance the stability and degree of supercoiling of the DNA, either alone or in combination with the TE buffer. Stabilizers such as lipids and carbohydrates can also increase the overall amount of DNA encapsulated into the microparticles. Combinations of stabilizers can be used to increase the amount of encapsulated, supercoiled DNA. Stabilizers such as charged lipids (e.g., CTAB), cationic peptides, or dendrimers (*J. Controlled Release,* 39:357, 1996) can condense or precipitate the nucleic acid (e.g., DNA). Moreover, stabilizers can have an effect on the physical nature of the particles formed during the encapsulation procedure. For example, the presence of sugars, lipids, or surfactants during the encapsulation procedure can generate porous or hollow particles with porous interior or exterior structures, allowing for a more rapid exit of a drug from the particle, rather than a slow controlled release over several days or months. The stabilizers can act at any time during the preparation of the microparticles (e.g., during encapsulation or lyophilization, or both) or during the degradation of the polymer *in vivo.*

The microparticles of the invention are generally formulated in one of two ways: (1) to maximize delivery into the patient's phagocytic cells, or (2) to form a deposit in the tissues of the patient, from which the nucleic acid is released gradually over time; upon release from the deposit, the nucleic acid is taken up by neighboring cells (including antigen presenting cells, or APCs). In both cases, maintaining the integrity of the DNA is a priority. For plasmid DNA. this means maximizing the percentage of plasmid molecules that are supercoiled and which may be capable of more efficient transfection and transcription than non-supercoiled (i.e., nicked or linear) plasmids. Maximizing the percentage of supercoiled plasmid molecules may also increase the stability of the DNA in the cell or microparticle, as well as the shelf life of the nucleic acid-containing microparticles.

Means for protecting the integrity of the nucleic acid include minimizing the shearing forces to which the nucleic acid is necessarily exposed in the process of microparticle formation, limiting sonication, homogenization, microfluidization, or other mixing times during preparation, controlling lyophilization, drying, or hardening, adding buffers or other stabilizers during microparticle preparation, and limiting the time that nucleic acid is exposed to high temperatures (e.g., limiting exposure to temperatures above about 39°C to less than about an hour). For example, it is desirable to achieve a balance between homogenization time and intensity which minimizes shear yet produces the desired size of microparticles with an acceptably high encapsulation efficiency (i.e, an encapsulation efficiency of ≥ 25% of the nucleic acid). These techniques are discussed below.

The microparticles of the invention can be used in the manufacture of a medicament for the treatment of, for example, cancer, infectious disease, any of the autoimmune diseases listed in Table 1, or any other condition treatable with a particular defined nucleic acid.

Phagocytosis of microparticles by macrophages, dendritic cells, and other APCs is an effective means for introducing the nucleic acid into these cells. Phagocytosis by these cells can be increased by maintaining a particle size below about 20 µm, preferably below about 11 µm, and most preferably below about 5 µm. The type of polymer used in the microparticle can also affect the efficiency of uptake by phagocytic cells, as discussed below.

The microparticles can be delivered directly into the bloodstream (i.e.. by intravenous or intraarterial injection or infusion) where uptake by the phagocytic cells of the reticuloendothelial system (RES) is desired. Alternatively, the microparticles can be delivered orally (e.g., to Peyers patches or mesenteric lymph nodes, mucosally, nasally, buccally, vaginally, rectally or intralesionally). The microparticles can also be delivered via subcutaneous injection, to facilitate take-up by the phagocytic cells of the draining lymph nodes. Alternatively the microparticles can be introduced intradermally (i.e., to the APCs of the skin, such as dendritic cells and Langerhans cells) or intramuscularly. Finally, the microparticles can be introduced into the lung (e.g., by inhalation of powdered microparticles or of a nebulized or aerosolized solution or suspension containing the microparticles), where the particles are picked up by the alveolar macrophages.

Once a phagocytic cell phagocytoses the microparticle, the nucleic acid is released into the interior of the cell. Upon release, it can perform its intended function: for example, expression by normal cellular transcription/translation machinery (for an expression vector), or alteration of cellular processes (for antisense or ribozyme molecules, or CpG or poly-I:C containing nucleic acids).

Because these microparticles are passively targeted to macrophages and other types of professional APC and phagocytic cells, they represent a means for modulating immune function. Macrophages and dendritic cells serve as professional APCs, expressing both MHC class 1 and class II molecules. In addition, the mitogenic effect of DNA can be used to stimulate non-specific immune responses mediated by B, T, and NK cells; macrophages; and other cells.

Delivery, via microparticles, of an expression vector encoding a foreign antigen which binds to an MHC class I or class II molecule will induce a host T cell response against the antigen, thereby conferring host immunity.

Where the expression vector encodes a blocking peptide (See, e.g., WO 94/04171) that binds to an MHC class II molecule involved in autoimmunity, presentation of the autoimmune disease-associated self peptide by the class I molecule is prevented, and the symptoms of the autoimmune disease alleviated.

In another example, an MHC binding peptide that is identical or almost identical to an autoimmunity-inducing peptide can affect T cell function by tolerizing or anergizing the T cell. Alternatively, the peptide could be designed to modulate T cell function by altering cytokine secretion profiles following recognition of the MHC/peptide complex. Peptides recognized by T cells can induce secretion of cytokines that cause B cells to produce antibodies of a particular class, induce inflammation, and further promote host T cell responses.

Induction of immune responses, e.g., specific antibody responses to peptides or proteins, can require several factors. It is this multifactorial nature that provides impetus for attempts to manipulate immune related cells on multiple fronts, using the microparticles of the invention. For example, microparticles can be prepared which carry both DNA and peptides, polypeptides, and/or adjuvants within each microparticle; alternatively, separate batches of microparticles can be prepared each of which carries only one of these substances, and then mixed. These dual-function microparticles are discussed below.

### CTL Responses

Class I molecules present antigenic peptides to immature T cells. To fully activate T cells, factors other than the antigenic peptide are required. Non-specific proteins such as interleukin-2 (IL-2), IL-12, and gamma interferon (γ-IFN) promote CTL responses and can be provided together with DNA encoding polypeptides which include CTL epitopes. Alternatively, proteins which bear helper T (Th) determinants can be included with DNA encoding the CTL epitope or epitopes. T helper epitopes promote secretion of cytokines from T helper cells and play a role in the differentiation of nascent T cells into CTLs.

Alternatively, peptides, proteins, nucleic acids, or adjuvants which promote migration, differentiation, or proliferation of lymphocytes and macrophages to a particular area could be included in microparticles, along with appropriate DNA molecules. Uptake of the DNA is enhanced as a result, because release of the protein would cause an influx of phagocytic cells and T cells as the microparticle degrades. The macrophages would phagocytose the remaining microparticles and act as APC, and the T cells would become effector cells.

### Antibody Responses

Elimination of certain infectious agents from the host may require both antibody and CTL responses. For example, when influenza virus enters a host, antibodies can often prevent it from infecting host cells. However, if cells are infected, then a CTL response is required to eliminate the infected cells and to prevent the continued production of virus within the host.

In general, antibody responses are directed against conformational determinants and thus require the presence of a protein or a protein fragment containing such a determinant. In contrast, T cell epitopes are linear determinants, typically just 7-25 residues in length. Thus, when there is a need to induce both a CTL and an antibody response the microparticles can include both an antigenic protein and the DNA encoding a T cell epitope. Slow release of the protein from microparticles outside of cells would lead to B cell recognition and subsequent secretion of antibody, while phagocytosis of the microparticles would cause APCs (1) to express the DNA of interest, thereby generating a T cell response; and (2) to digest the protein released from the microparticles, thereby generating peptides which are subsequently presented by class I or II molecules. Presentation by class I or II molecules promotes both antibody and CTL responses, since T helper cells activated by the class II/peptide complexes would secrete non-specific cytokines.

### Microparticles for Implantation

A second microparticle formulation of the invention is intended not to be taken up directly by cells, but rather to serve primarily as a slow-release reservoir of nucleic acid that is taken up by cells only upon release from the microparticle. Release may occur, for example, by degradation, erosion, or diffusion. The nucleic acid can be complexed to a stabilizer, e.g., to maintain the integrity of the nucleic acid during the slow-release process. The polymeric particles in this embodiment should therefore be large enough to minimize the extent of phagocytosis (i.e., larger than 5 µm and preferably larger than 20 µm). Such particles are produced by the methods described for making the smaller particles, but with less vigorous mixing. That is to say, a lower homogenization speed can be used to obtain particles having a diameter around 100 µm rather than 5 µm. The time of mixing, the viscosity of the first emulsion, and the concentration of polymer in the first solution can also be altered to affect particle dimension.

The larger microparticles can be formulated as a suspension, a powder, or an implantable solid, to be delivered by intramuscular, subcutaneous, intradermal, intravenous, or intraperitoneal injection; via inhalation (intrapulmonary); orally, e.g. in the form of a tablet; intranasally or by implantation. These particles are useful for delivery of any expression vector or other nucleic acid for which slow release over a relatively long term is desired: e.g., an antisense molecule a gene replacement therapeutic, a means of delivering cytokine-based antigen-based or hormone-based therapeutic, or an immunosuppressive agent. The rate of degradation, and consequently of release, varies with the polymeric formulation. This parameter can be used to control immune function. For example, one might want a relatively slow release for delivery of IL-4 or IL-10. and a relatively rapid release for delivery of IL-2 or γ-IFN.

### Composition of Polymeric Particles

Polymeric material is obtained from commercial sources or can be prepared by known methods. For example, polymers of lactic and glycolic acid can be generated as described in US Patent No. 4,293,539 or purchased from a commercial source such as Aldrich Chemicals. Birmingham Polymers, or Boehringer lngelheim. One suitable polymer is poly-lactic-*co-*glycolic acid) (PLGA), with a lactic/glycolic acid weight ratio of about 1:2 to about 4:1 (e.g., 50:50,65:35, or 75:25).

Alternatively, or in addition, the polymeric matrix can include any one or more of polylactide, polyglycolide, polyanhydride, polyorthoester, polycaprolactone, polyphosphazene, polypeptide, polyester, or naturally occurring polymers such as alginate, chitosan, and gelatin.

Preferred controlled release substances which are useful in the formulations of the invention include the polyanhydrides, co-polymers of lactic acid and glycolic acid wherein the weight-ratio of lactic acid to glycolic acid is no more than 4:1. and polyorthoesters containing a degradation-enhancing catalyst, such as an anhydride, e.g., 1% maleic anhydride. Since polylactic acid can take at least one year to degrade *in vivo*, this polymer should be utilized by itself only in circumstances where extended degradation is desirable.

In some cases, the polymeric matrix also includes a targeting molecule such as a ligand, receptor, or antibody, to increase the specificity of the microparticle for a given cell type or tissue type.

### Preparation of Microparticles:

Microparticles are prepared by making a first emulsion (i.e., via a batch process or a continuous flow process) from an aqueous solution containing a nucleic acid and a solution containing a polymeric material dissolved in an organic solvent (e.g., dichloromethane, phenol, chloroform, or ethyl acetate), and then combining the first emulsion with a surfactant-containing second aqueous solution (e.g., a solution of poly(vinyl alcohol), PVA; oleic acid; TWEEN®; SPAN®; poly(vinylpyrrolidone), PVP; other surface-reactive agents) to produce a second emulsion from which the microparticles are isolated. The microparticles are then purified and optionally concentrated, for example, by diafiltration, or using a Sweco™ device (Emerson Electric Co., Florence, KY).

Diafiltration is a process by which particles and relatively high molecular weight solutes can be separated from relatively low molecular weight solutes, where both are present in the same suspension. The suspension is diluted with solvent (e.g.. water) and passed through a hollow fiber membrane, washing away the relatively low molecular weight solutes from the retentate. In the present methods, diafiltration can be used to remove excess surfactants (e,g., PVA), stabilizers (e.g., sucrose), and buffers (e.g., TE) from the microparticles.

Sweco devices such as the PhamiASep Filter-Dryer™ (Emerson Electric Co.. Florence, KY) can be used in place of diafiltration. A liquid slurry of the microparticles is pumped into the Sweco device for purification. As the liquid passes through the device, the solid microparticles are restrained by a vibrating fine mesh screen. The microparticles can be washed with water-for-injection to remove soluble contaminants (e.g., PVA. sucrose). Optionally, the microparticles can also be coated with an excipient in the device (i.e., by washing with a solution of the excipient). Examples of excipients are PEG, carboxymethylcellulose, sorbitol, TWEEN, and mannitol. A flow of sterile air or nitrogen is then passed through the device to dry the microparticles. The dried microparticles, in the form of a powder are discharged through an exit port into a sterile receiving vessel. A powder augur is then used to carry the powder up a tube at a measured rate and into individual vials. Alternatively, microparticles can be washed to remove solvent, concentrated using a vibrating or non-vibrating stainless steel mesh screen device, and then dried (e.g., in a lyophilizer).

In the processes of the invention, the first emulsion and the surfactant-containing solution are pumped into a mixing chamber, including, for example a microfluidizer (e.g., as available from Microfluidics, Newton, MA), a French press (e.g., as available from Microfluidics or Gaulin-APV), a mechanical homogenizer (e.g. as available from Silverson, VirTis, or Ika Works), or an ultrasonic emulsifier (e.g., as available from Branson Ultrasonics Corporation or VirTis). In the mixing chamber, the first emulsion and the surfactant-containing solution are blended to form a second emulsion. The surfactant-containing solution can optionally include a stabilizer, such as a nucleic acid release modulator, a buffer, a carbohydrate or a lipid. It is important that the second aqueous solution has an osmolarity, pH, and buffering capacity essentially identical to that of the first aqueous solution to minimize loss of the nucleic acid into the aqueous phase of the second emulsion.

As the first emulsion and surfactant-containing solution are pumped info the mixing chamber, the second emulsion can be continuously pumped out of the mixing chamber into a solvent removal device. The solvent removal device may be any vessel (e.g., a closed vessel) that can be sterilized. Examples of suitable solvent removal devices include pharmaceutical pressure vessels (e.g., as available from Eagle Stainless Container, Alloy Products Corporation, DCI Inc., Walker Stainless Equipment), bioreactors/fermenters (e.g., as available from Applikon. New Brunswick, Chemap, B. Braun, and LH), and hollow-fiber membrane devices. In the solvent removal device, the organic solvent is removed (e.g., by evaporation with heating (e.g., to 30-55°C) or an applied vacuum; by extraction; by addition of an alcohol such as isopropanol or ethanol; by dilution with water, or using hollow fiber membranes.

During removal of the organic solvent, microparficliig containing nucleic acid encapsulated in a polymeric matrix or shell precipitate from the emulsion forming a suspension. Following removal of the organic solvent the microparticle-containing suspension can be continuously removed from the solvent removal device into a filtration or wash reservoir. The contents of the reservoir are diluted with an aqueous wash solution (e.g., water) and pumped into a filtration apparatus (e.g., a diafiltration apparatus). Aqueous solution is removed from the apparatus, leaving the microparticles behind. The removed solution includes, for example any excess surfactant that is not incorporated into the microparticles. The suspension of microparticles can be concentrated by removing the aqueous solution from the filtration apparatus at a higher rate than the wash solution is pumped in, or by ceasing the inflow of the wash solution altogether. The final, concentrated suspension can contain excipients such as mannitol. PEG, carboxymethylcellulose, sorbitol, and TWEEN. The suspension can then be aliquotted into single dose vials. Freezing and lyophilizing the filtered microparticle suspension in a single dose vial results in an easily stored dry preparation. Alternatively, the solution containing the particles can be pumped onto a fine mesh sieve such as a Sweco device for washing and drying. The sieve would allow the aqueous solution to pass trough while retaining the particles. Particles washed on a Sweco unit would be dried by passing sterile air over the particles. These particles would-be removed from the Sweco unit into a holding vessel. From here the particles could be aliquotted into single dose vials by way of a rotating screw that deposits a predetermined amount of particles into each vial. The vials would then be sealed for storage. Particles may also be retained in a Sweco device for washing, and then dried in a lyophilizer before being aliquotted into single dose vials.

It is important to generate microparticles with very little residual organic solvent, so that the particles are suitable for use in animals. The amount of residual organic solvent in the dried or lyophilized particles is preferably below 200 ppm, and more preferably below 50 ppm,

It is also important that the particles be prepared in an aseptic fashion (e.g., as defined in U.S. Pharmacopeia USP24<71>) if they are to be used in the treatment of disease in animals (e.g., humans). The processes described herein have been designed such that sterility can be maintained throughout and the resulting product is essentially free of biological contaminants. Techniques for sterilizing machinery and filtering solutions aseptically are known by those skilled in the art of pharmaceutical manufacturing (e.g., good manufacturing practice ("GMP") manufacturers of biological products), and are employed in the present processes.

Larger particles such as those used for implantation, can be obtained by using less vigorous emulsification conditions when making the second emulsion, by altering the concentration of the polymer, altering the viscosity of the emulsion altering the particle size of the first emulsion (e.g., larger particles can be made by decreasing the pressure used while creating the first emulsion in a microfluidizer), or homogenizing with, for example, the Silverson homogenizer set at 5000 rpm for about 12 seconds.

The washed, or washed and lyophilized, or washed and dried microparticles can be suspended in an excipient without negatively affecting the amount of supercoiled plasmid DNA within the microparticles. Excipients such as saline, carbohydrates, detergents and other surfactants, polymers, buffers, and lipids are often used in drug formulation, and here provide for efficient microparticle resuspension, act to prevent settling, and/or increase *in vivo* dispersion. According to analysis by gel electrophoresis, excipients such as TWEEN 80. mannitol, sorbitol, and carboxymethylcellulose do not have a deleterious effect on nucleic acid stability or supercoiling, when included prior to or after lyophilization.

### Characterization of Microparticles

The size distribution of the microparticles prepared by the above method can be determined with a COULTER™ counter. This instrument provides a size distribution profile and statistical analysis of the particles. Alternatively, the average size of the particles can be determined by visualization under a microscope fitted with a sizing slide or eyepiece.

If desired, the nucleic acid can be extracted from the microparticles for analysis by the following procedure. Microparticles are dissolved in an organic solvent such as chloroform or methylene chloride in the presence of an aqueous solution. The polymer stays in the organic phase, while the nucleic acid goes to the aqueous phase. The interface between the phases can be made more distinct by centrifugation. Isolation of the aqueous phase allows recovery of the nucleic acid. The nucleic acid is retrieved from the aqueous phase by precipitation with salt and ethanol in accordance with standard methods. To test for degradation, the extracted nucleic acid can be analyzed by HPLC. capillary gel electrophoresis, or agarose gel electrophoresis.

The amount of residual organic solvent in the particles can be determined by methods known in the art, such as, for example, gas chromatography or gas chromatography coupled to mass spectrometry. More specifically, microparticles were produced by the procedure detailed in Example 2 and were tested for residual solvent as follows:

Four vials representing various stages of the vial fill were sequestered for analysis. Approximately 15 mg of lyophile was removed from each of the four vials and combined with gentle mixing (using a hand-held spatula) to ensure homogeneity of the test article. The pooled lyophile was then resuspended in tetrahydrofuran with an internal standard (1.2-dichloropropane), to adjust for variations in injection volume from injection to injection. Each test article was subjected to duplicate injections into a gas chromatograph outfitted with a Supelco™ SPB-5 30m X 0.53mm column and an electron capture detector. Peak areas of dichloromethane DCM and injection control were determined (both peaks achieve baseline resolution). The DCM/1,2-dichloropropane ratio was determined for the test article and was compared to the corresponding ratio from a standard prepared to a known concentration. This comparison yielded the concentration of DCM in the test article. The test method is quantitative in the 50 to 100 ppm DCM concentration range, with a limit of quantitation of approximately 30 ppm and a limit of detection of approximately 20 ppm. The procedure was applied to two preparations of microparticles. One preparation was found to have a residual DCM level at around the limit of detection (i.e., about 20 ppm). The other had a residual DCM level of 79 ppm.

Non-GMP samples were analyzed in a similar manner. Lyophilized test article was resuspended in THF with 1,2-dichloropropane and subjected to GC analysis as previously described. The liquid test article was injected directly into the GC without benefit of sample preparation. A DCM standard curve was generated for each sample set and DCM concentrations of test articles are extrapolated from the standard curve.

### In Vivo Delivery of Microparticles

Microparticles containing nucleic acid can be resuspended in saline, buffered salt solution, tissue culture medium, carbohydrate- or lipid-containing solution, or other physiologically acceptable carrier. They can be injected into an animal (e.g., a mammal such as a human) intramuscularly, intravenously, intraarterially, intradermally, intrathecally, intraperitoneally, or subcutaneously, or they can be introduced into the gastrointestinal tract or the respiratory tract, e.g., by inhalation of a suspension or powder containing the microparticles, or swallowing a tablet or suspension containing the microparticles. Alternatively the microparticles can be introduced into a mucosal site such as the vagina, nose, or rectum, Expression of the nucleic acid is monitored by an appropriate method. For example, expression of a nucleic acid encoding an immunogenic protein of interest is assayed by RT-PCR or by looking for an antibody or T cell response to the protein.

Antibody responses can be measured by testing serum in an ELISA assay. In this assay, the protein of interest is coated onto a 96 well plate and serial dilutions of serum from the test subject are pipetted into each well. A secondary, enzyme-linked antibody, such as anti-human, horseradish peroxidase-linked antibody, is then added to the wells. If antibodies to the protein of interest are present in the test subject's serum, they will bind to the protein fixed on the plate, and will in turn be bound by the secondary antibody. A substrate for the enzyme is added to the mixture and a colorimetric change is quantitated in an ELISA plate reader. A positive serum response indicates that the immunogenic protein encoded by the microparticle's DNA was expressed in the test subject, and stimulated an antibody response. Alternatively, an ELISA spot assay can be employed.

T cell proliferation in response to a protein following intracellular delivery of microparticles containing nucleic acid encoding the protein is measured by assaying the T cells present in the spleen, lymph nodes, or peripheral blood lymphocytes of a test animal. The T cells obtained from such a source are incubated with syngeneic APCs in the presence of the protein or peptide of interest. Proliferation of T cells is monitored by uptake of ³H-thymidine, according to standard methods. The amount of radioactivity incorporated into the cells is directly related to the intensity of the proliferative response induced in the test subject by expression of the microparticle-delivered nucleic acid. A positive response indicates that the microparticle containing DNA encoding the protein or peptide was taken up and expressed by APCs *in vivo*.

The generation of cytotoxic T cells can be demonstrated in a standard "Cr release assay (see, e.g.. PCT application WO99/18995, USSN 09/398.534. or USSN 60/154,665). In these assays, spleen cells or peripheral blood lymphocytes obtained from the test subject are cultured in the presence of syngeneic APCs (e.g., dendritic cells) and either the protein of interest or an epitope derived from this protein. After a stimulation cycle (e.g., 4-10 days), the effector cytotoxic T cells are mixed with ⁵¹Cr-labeled target cells expressing an epitope derived from the protein of interest. Effector cells can be stimulated in vitro 1-3 times. If the test subject raised a cytotoxic T cell response to the protein or peptide encoded by the nucleic acid contained within the microparticle, the cytotoxic T cells will lyse the targets. Lysed targets will release the radioactive ⁵¹Cr into the medium. Aliquots of the medium are assayed for radioactivity in a scintillation counter. Assays, such as ELISA or FACS, can also be used to measure cytokine profiles of responding T cells (see, e.g., PCT application WO99/18995, USSN 09/398,534, or USSN 60/154,665).

### Lipid-Containing Microparticles

The microparticles described herein can also include one or more types of lipids as stabilizing compounds. The inclusion of a lipid in a microparticle can increase the stability of the nucleic acid in the microparticle, e.g., by maintaining a covalently closed double-stranded DNA molecule in a supercoiled state. The presence of a lipid in the particle can modulate. i.e.. increase or decrease, the rate at which a drug or nucleic acid is released from the microparticle.

Addition of a lipid to the microparticle can in certain cases increase the efficiency of encapsulation of the nucleic acid or increase the loading of the nucleic acid within microparticles. For example, the encapsulation efficiency may be improved because the presence of the lipid reduces the surface tension between the inner aqueous phase and the organic phase. Reduction of the surface tension is thought to create an environment more favorable for the nucleic acid, and therefore to increase its retention within the microparticle. A reduction in surface tension also allows for the primary emulsion to be formed with less manipulation, which minimizes shearing of the nucleic acid and increases supercoiling. It is also possible that the presence of lipid in the microparticle may enhance the stability of the microparticle/nucleic acid formulation, and may increase the hydrophobic nature of the microparticles, thereby increasing uptake by phagocytic cells.

The lipids can be cationic, anionic, or zwitterionic, or may carry no charged groups, such as nonpolar glycerides. They can be, for example, fatty acids, eicosanoids, glycerophospholipids, triacetylglycerols, waxes, sphingolipids, steroids (e.g.. cholesterol. CHAPS. bile acids, hormones, cardiac aglycones), lipid vitamins, or terpenes. The lipids preferably are not present as liposomes that encapsulate (i.e.. surround) the microparticles. The lipids may optionally form micelles.

Examples of lipids that can be used in the microparticles include acids (e.g., carboxylic acids, including fatty acids such as capric acid), bases (such as amines), zwitterionic lipids (e.g.. CHAPS), phospholipids such as phosphatidylethanolamine, phosphatidyl glycerol, phosphatidyl serine, phosphatidyl inositol, phosphatidylcholine, or phosphatidic acid, each containing one or more of the following, groups: propionyl (propanoyl), butyryl (butanoyl), valeryl (pentanoyl) caproyl (hexanoyl), caprylyl (heptanoyl), capryl (decanoyl), undecanoyl, lauryl (dodecanoyl), tridecanoyl, myristyl (tetradecanoyl), pentadecanoyl, palmityl (hexadecanoyl), phytanoyl (3,7,11,15-tetramethylhexadecanoyl), heptadecanoyl, stearyl (octadecanoyl), bromostearyl, nonadecanoyl, arachidoyl (eicosanoyl), heneicosanoyl, behenyl (docosanoyl), tricosanoyl, lignoceryl (tetracosanoyl), myristoleoyl (9-*cis*-tetradecanoyl), myristelaidoyl (9-*trans-*tetradecanoyl), palmitoleyl (9-*cis*-hexadecanoyl), palmitelaidyl (9-*trans*-hexadecenoyl), petroselinoyl (6-*cis*-octadecenoyl), oleoyl (9-*cis*-octadecenoyl), elaidoyl (9-*trans*-octadecenoyl), linoleoyl (9-*cis*-12-*cis*-octadecadienoyl), linolenoyl (9-*cis*-12-*cis*-15-*cis*-octadecatrienoyl), eicosenoyl (11-*cis*-eicosenoic), arachidonyl (5,8,11,14-(all-*cis*)-eicosatetraenoic), erucoyl (13-*cis*-docosenoic), and nervonoyl (15-*cis*-tetracosenoic).

Other suitable lipids include the cetyltrimethyl ammonium ion, which is available as CTAB, PEG-DSPE, and those that contain steroid structures such as cholesterol, CHAPS, and certain vitamins and hormones. Saponin-derived lipids (e.g., QS21) may also be used.

Mixtures of lipids can be used to make a lipid-containing microparticle. Suitable commercially available lipid preparations include lecithin, OVOTHIN 160™, and EPIKURON 135F™ lipid suspensions, all of which are available from Lucas Meyer, Inc., Decatur, IL.

The lipid may also be isolated from an organism, e.g., a mycobacterium. The lipid is preferably a CD1-restricted lipid, such as the lipids described in Pamer, *Trend Microbiol.* 7:13, 1999; Braud, *Curr Opin. Immunol.* 11:100, 1999; Jackman, *Crit. Rev. Immunol*.19:49, 1999; and Prigozy, *Trends Microbiol.* 6:454, 1998.

Instead of, or in addition to, incorporating lipids into the microparticles, the microparticles can be suspended in a lipid (or lipid suspension) to improve dispersion, delivery following injection, or to keep them in a suspended state.

The lipid-containing microparticles may also include additional stabilizers, as described above. The inclusion of a lipid in a microparticle along with a stabilizer such as sucrose can provide a synergistic increase in the release of nucleic acids within the microparticle.

As described above, lipid-containing microparticles can be prepared by adding a lipid to the organic solvent containing the polymer, to the aqueous solution containing the DNA solution, or to the aqueous solution containing the surfactant. The solubility properties of a particular lipid in an organic or aqueous solvent will determine which solvent is used.

Some lipids or lipid suspensions can be added to either the organic solvent or the aqueous solution. Microparticles may in addition be resuspended in a lipid-containing solution to facilitate resuspension and dispersion of the microparticles.

In addition to the lipid-containing microparticles described herein, microparticles may also be made by incorporating into the polymer solution (i.e., along with PLGA) other macromolecules such as chitin, gelatin, or alginate, or various combinations of these macromolecules and lipids. Microparticles made with these other macromolecules may in addition include the above-described stabilizing agents.

The method is illustrated by the following examples.

### EXAMPLES

### EXAMPLE 1: Preparation of DNA

500 ml bacterial cultures containing plasmid DNA were poured into one liter centrifuge bottles. The cultures were centrifuged at 4000 rpm at 20°C for 20 minutes. The medium was removed from the pelleted bacteria. The bacterial pellet was completely resuspended in 50 ml buffer P1 (50 mM Tris-HCl, pH 8.0; 10 mM EDTA; 100 µg/ml RNase), leaving no clumps. 50 ml of buffer P2 (200 mM NaOH, 1% SDS) was added with gentle swirling, and the suspensions were incubated at room temperature for five minutes to lyse the cells. 50 ml of buffer P3 (3.0 M potassium acetate, pH 5.5, chilled to 4°C) was added with immediate, gentle mixing. The suspensions were incubated on ice for 30 minutes, then centrifuged at 4000 rpm at 4°C for 30 minutes.

A folded, round filter was wetted with water. When the centrifugation was complete the supernatant was immediately poured through the filter. The filtered supernatant was collected in a clean 250 ml centrifuge bottle.

15 ml of Qiagen ER™ buffer was added to the filtered lysate, mixing by inverting the bottle 10 times. The lysate was incubated on ice for 30 minutes.

A Qiagen-tip 2500™ column was equilibrated by applying 35 ml Qiagen QBT™ buffer (750 mM sodium chloride; 50 mM MOPS, pH 7.0; 15% isopropanol; and 0.15% Triton X-100). The column was allowed to empty by gravity flow. The incubated lysate was applied to the column and allowed to enter by gravity flow. The column was washed with 4 x 50 ml Qiagen Endofree QC™ buffer (1.0 M NaCl: 50 mM MOPS, pH 7.0; 15% isopropanol). The DNA was eluted from the column with 35 ml of QN™ buffer (1.6 M NaCl; 50 mM MOPS, pH 7.0; 15% isopropanol) into a 50 ml polypropylene screwcap centrifuge tube. The DNA suspension was split into two tubes by pouring approximately 17.5 ml of the suspension into a second 50 ml screwcap tube.

Using a sterile 10 ml pipette, 12.25 ml isopropanol was added to each tube. The tubes were closed tightly and thoroughly mixed. The contents of each tube were poured into 30 ml Corex™ (VWR) centrifuge tubes. Each Corex tube was covered with PARAFILM®. The tubes were centrifuged at 11,000 rpm at 4°C for 30 minutes.

The supernatant was aspirated from each tube and the pellet was washed with 2 ml 70% ethanol. The ethanol was aspirated off. The pellet was air dried for 10 minutes, then resuspended in 0.5-1.0 ml water and transferred to a sterile 1.5 ml microfuge tube.

### EXAMPLE 2: Preparation of Microparticles

Referring to Fig. 1, a bottle 5 containing 50 ml of dichloromethane (DCM) into which had been dissolved 6 g of poly-lactic-*co*-glycolic acid ("PLGA/DCM"), was placed in an ice tub. The bottle 5 was fitted with a Silverson SL2T™ Homogenizer 7.

10.8 ml of a DNA/sucrose/TE solution (i.e., 72 mg DNA, 300 mM sucrose, and 10 mM TE) was injected into the PLGA/DCM solution using a syringe. Homogenization at 10,000 rpm was begun concurrently with the injection of the DNA solution, and continued for 15 minutes.

An additional 100 ml of DCM was then injected into the resulting emulsion with a syringe over a period of not more than 20 seconds. After the injection of the 100 ml DCM. the homogenization was allowed to continue for an additional 30 seconds, and homogenizer 7 was then stopped. Bottle 5 containing the first emulsion was then removed from the ice tub and attached to conduit 14. Conduit 14 was routed through pump 16 to homogenizing chamber 23 fitted with a Silverson L4RT™ Homogenizer 24.

Microparticles were prepared by homogenizing the first emulsion with a surfactant. The mixing chamber 23 was in line with a reservoir 18 containing 1% polyvinyl alcohol (PVA)/10.37% sucrose solution. The reservoir 18 was connected via conduit 20 through pump 22 to the mixing chamber 23.

The PVA/sucrose solution was drawn from reservoir 18 through pump 22 set at a flow rate of 1 l/min. Immediately after beginning the flow of the PVA/sucrose solution, the homogenizer 24 was set to 6,000 rpm. A second emulsion was then formed by drawing the first emulsion from bottle 5 into chamber 23 by setting pump 16 to a flow rate of 36 ml/min. After homogenization, the second emulsion exited through conduit 25 and entered a BIOFLOW 2000® bioreactor chamber 28 (New Brunswick Scientific, Edison. NJ) containing a stirrer operating at 50 rpm. Conduit 25 was fitted with clamp 26 to regulate flow of the second emulsion between homogenizer 24 and bioreactor chamber 28.

Bioreactor chamber 28 also featured inlet 32 for delivering nitrogen, pressure relief valve 34, and outlet conduit 36 to which was attached clamp 38. Conduit 36 was in communication with "Y"-connector 40, which diverted flow from conduit 36 into conduit 42. Fluid flow through conduit 42 was regulated by clamp 44. Alternatively, fluids could be introduced into conduit 48 via conduits 41 and 42 by closing clamp 38 and opening clamps 43 and 44.

• Five liters of the PVA/sucrose solution from reservoir 18 and 150 ml of the first emulsion from bottle 5 were pumped into homogenizer 24, and then into bioreactor chamber 28. over a five minute period. After the solutions had been pumped through the homogenizer 24. the stirring speed in the bioreactor chamber 28 was increased to 375 rpm for 30 minutes.

The temperature of the bioreactor chamber 28 was set to 22°C and monitored every 5 minutes. After 30 minutes, the temperature set point was increased to 37°C, and the temperature was again monitored every 5 minutes. When the temperature had reached 37°C. the stirring speed was maintained at 375 rpm and stirring was continued for 1.5 hours, after which time the stirring speed was adjusted to 150 rpm and the temperature set point was decreased to 15°C.

Washing of the microparticles was initiated by introducing 500 ml of water (4°C) through conduits 41 and 42 (i.e., with clamp 38 closed), pump 46. and conduit 48, to an AG Technologies hollow fiber reservoir 50. The flow rate through hollow fiber reservoir 50 was initially set at a rate of 11 l/min via pump 57.

After addition of the 500 ml water to hollow fiber reservoir 50. clamp 43 was closed, and clamp 38 released. Pump 46 was then used to transfer solution from bioreactor chamber 28 to hollow fiber reservoir 50 at a flow rate of 80 ml/min.

Hollow fiber reservoir 50 was connected to a pressure release vent 52. which had a filter 54, and to conduits 55 and 56. Fluid was circulated from the hollow fiber reservoir 50 through conduit 56 via pump 57 to serially stacked hollow fiber cartridges 59, each having polysulfone porous membranes with 0.2 µm pores. Fluid entering hollow fiber cartridges 59 from conduit 56 passed into the interior region of the hollow fibers. This region, in turn, was in communication with conduit 64. The size of the hollow fiber membrane's pores was sufficiently small so that the microparticles could not pass through the membranes. In contrast, reagents and byproducts of the manufacturing process, e.g., the PVA and sucrose, did pass through the membranes. This permeate was removed through conduit 62 by pump 66 using a flow rate of 40 ml/min. The total flow rate was thus 80 ml/min for the two chambers.

Conduit 56 from hollow fiber reservoir 50 was also connected to clamp 70, which regulated flow of liquid into a 600 ml glass beaker 72. As explained in more detail below, release of clamp 70 allowed for delivery of microparticles from the hollow fiber reservoir 50 to beaker 72.

With clamp 70 closed, fluid was directed from hollow fiber reservoir 50 through conduit 56 by pump 57 into hollow fiber cartridges 59. Fluid was allowed to flow until less than 1 liter of fluid remained in bioreactor chamber 28. At this point, the flow rate was increased so that all of the solution was transferred from bioreactor chamber 28 to hollow fiber reservoir 50 over 2 minutes. The nitrogen flow to bioreactor chamber 28 was then stopped, as was stirring within bioreactor chamber 28.

The microparticles were washed by pumping cold water (4°C) into the hollow fiber system through conduit 48 via conduits 41 and 42, using pump 46 at a flow rate of 80 ml/min. The volume of solution in the hollow fiber reservoir 50 was kept constant at 500 ml. Washing was continued until 3.5 liters of cold water (4°C) had been pumped into the system. At this point, pump 46 was stopped. Pump 66 continued to carry out permeate at 80 ml/min until approximately 200 ml of solution remained in the hollow fiber reservoir 50. Pumps 46 and 57 were then stopped, and clamp 70 was opened to allow microparticles to flow from the hollow fiber reservoir 50 to beaker 72.

Clamp 70 was then closed and 100 ml of cold water (4°C) was introduced into the system from conduit 41 using pump 46, to rinse hollow fiber cartridges 59 so as to recover microparticles left behind in the first collection described above and to thereby maximize product yield. Pump 57 was then turned on, and the recirculating flow rate was slowly increased to 11 l/ml and maintained for 5 minutes, then decreased by gradually decreasing the flow rate, as controlled by pump 57.

Clamp 70 was again opened to collect the microparticle suspension. The suspension was combined with the earlier collected microparticle suspension, and the weight of the combined microparticle suspensions was determined. The microparticles were stirred at room temperature for an additional time to further effect DCM removal.

The microparticle suspension was lyophilized after first aliquotting 1.5 ml into 5 ml vials. The vials were held at -50°C for 3 hours to freeze the suspension within the lyophilization chamber, after which the temperature was increased at the rate of 5°C per hour to a final temperature of -10°C. The vials were maintained at -10°C for a minimum of 6 minutes, and then at 25°C for 4-16 hours to lyophilize. The lyophilization chamber was backfilled with nitrogen gas to a pressure of~2" Hg, and rubber vial stoppers were seated while this pressure was maintained. When lyophilization was complete, the vials were crimped with rubber/aluminum septa, and stored at -20°C.

The particles were found to have a diameter of about 1-2 µm by number average.

### EXAMPLE 3: Results

The procedure of Example 2 was carried out with various nucleic acid loadings and conditions for preparing the first emulsion. The results were as follows:

### Trial #1

72 mg plasmid DNA
First emulsion prepared in microfluidizer
Yield: 4.6 g (77%)
Average microparticle diameter: 1.9 µm
Encapsulation efficiency: 4.8 µg DNA/mg microparticles
DNA remaining in supercoiled form: ≥60%
Residual PVA relative to the mass of microparticles: 0.88%

### Trial #2

72 mg plasmid DNA
First emulsion prepared in SL2T homogenizer, batch process
Yield: 4.5 g (74%)
Average microparticle diameter: 2.1 µm
Encapsulation efficiency: 5.09 µg DNA/mg microparticles
DNA remaining in supercoiled form: ≥60%
Residual PVA relative to the mass of microparticles: 0.89%

### Trial #3

72 mg plasmid DNA
First emulsion prepared in SL2T homogenizer (in this case, rather than 50 ml DCM and 10.8 ml DNA solution, all 150 ml DCM and all DNA solution was combined at once)
Yield: 5.0 g (83%)
Average microparticle diameter: 2.0 µm
Encapsulation efficiency: 3.99 µg DNA/mg microparticles
DNA remaining in supercoiled form: ≥80%
Residual PVA relative to the mass of microparticles: 1.1%

### Trial #4

108 mg plasmid DNA
First emulsion prepared in SL2T homogenizer
Yield: 5.6 g (93%)
Average microparticle diameter: 1.9 µm
Encapsulation efficiency: 7.04 µg DNA/mg microparticles
DNA remaining in supercoiled form: ≥60%
Residual PVA relative to the mass of microparticles: 1.48%

### Trial #5

108 mg plasmid DNA
First emulsion prepared in SL2T homogenizer,
Yield: 5.75 g (96%)
Average microparticle diameter: 1.8 µm
Encapsulation efficiency: 9.78 µg DNA/mg microparticles
DNA remaining in supercoiled form: 70%
Residual PVA relative to the mass of microparticles: 1.63%

### EXAMPLE 4: Alternative method for removal of residual organic solvent from the second emulsion

The microparticles are formed as described in Example 2. The secondary emulsion is pumped into a solvent removal device, wherein the DCM diffuses from the microparticles into the aqueous phase of the secondary emulsion. The slurry is agitated very gently in this tank; avoidance of vigorous agitation and aeration helps preclude the formation of foam. In contrast to the procedure described in Example 2. there is no gas overlay, but rather a partial vacuum is pulled over the headspace, venting any DCM that evaporates. As the DCM diffuses into the aqueous phase from the microparticles. DCM levels rise in the aqueous phase until reaching saturation (approximately 1-2%, dependent on temperature of the solution). During the hardening phase, additional water is added to the solvent removal device until a total of 10 liters of water is present per 150 ml DCM. The slurry is then transferred (e.g., by pressurizing the hardening tank) to a Sweco PharmaSep Filter/Dryer (Emerson Electric). This unit is fitted with a one-micron or less nominal screen size to retain microparticles greater than one micron in diameter. The screen size can be adjusted to retain microparticles of varying sizes. The product is retained inside the filter/dryer as the aqueous phase passes through the screen and is discharged.

Inside the Sweco unit, the microparticles are then washed slowly with water for injection (WFI), keeping the microparticles in suspension through screen vibration. The DCM passes from the microparticles into the water, which passes through the unit and carries the DCM away. Since the wash water entering the unit has no DCM, the microparticles are exposed only to very low DCM levels in the bulk aqueous phase, thereby speeding up the diffusive process for transferring DCM out of the microparticles. As DCM levels in the product drop, the flow rate of wash water can be reduced to conserve costs and aid solvent removal. The temperature of the wash water can be adjusted so as to maximize the rate of diffusive transfer without damaging the product. Finally, the product can be rinsed with a final excipient and dried with nitrogen gas to the appropriate state of dryness. The dried product is discharged from the filter/dryer into a sterile receiving container for vialing. Alternatively, the product can be retained in the filter device for washing, and then dried in a lyophilizer prior to discharge into a sterile receiving container for vialing.

If desired, the DCM can be recovered from the discharged wash water by distillation. Since the discharged wash water contains no product, it can be boiled and the offgas passed into a distillation column. DCM and water can be separated on the distillation column and the DCM can be recovered for re-use. This has the benefits of reducing the cost of DCM, which is generally lost in the current processes, and of eliminating the environmental problems associated with DCM vented to the atmosphere.

### EXAMPLE 5: 2X scale of the microparticle production process.

The 2X scaleup of the process calls for two primary homogenizers, one secondary (flow-through) homogenizer, two bioreactors, and one (2x-sized) hollow-fiber device.
1. A single emulsion was made using 6 g PLGA in 50 ml DCM and 72 mg of DNA in 10.8 ml of 10% sucrose in a 250-ml polypropylene custom container sealed by a TEFLON® stopper through which the emulsifier head penetrates. After 15 minutes of homogenization with a Silverson SR2T homogenizer at 10,000 rpm, an additional 100 ml of DCM was pumped into the container. The final volume of this emulsion was 161 ml. Homogenization proceeded for an additional 30 seconds.
2. Without removing the homogenizer tip, the emulsion was pumped out of the container through a stainless-steel tube that penetrated the TEFLON® stopper and reached the bottom of the container. The primary emulsion was pumped at 32.2 ml/min (5 minutes) along with 5 liters of PVA solution (1% PVA plus 10.37% sucrose), which was pumped at 1 liter/min (5 minutes) through a Silverson L4RT™ flow-through homogenizer into a 10-liter New Brunswick Scientific Bioflo™ reactor vessel. The reactor vessel was agitated at 375 rpm and sterile-filtered nitrogen gas was passed through the headspace at 20 liters/minute. The temperature of the reactor vessel was kept at 20°C for 30 minutes, then raised to 37°C and held at that temperature while continually stirring and gassing for an additional 2 to 3 hours.
3. While the first emulsion was stirring, step 1 was repeated with a second container and homogenizer.
4. Step 2 was repeated using the same secondary homogenizer and a different reactor vessel.
5. After the contents of first reactor vessel had stirred for 2 to 3 hours, the liquid in the reactor vessel was pumped at 8 l/min through a large hollow-fiber cartridge and recirculated back into the reactor vessel. The permeate was removed at a constant 160 ml/minute and discarded.
6. When one liter of permeate had been removed from the first reactor vessel, a set of valves was switched so as to put the second reactor vessel into the recirculation circuit. One liter of permeate was removed from the second reactor vessel and discarded.
7. Steps 5 and 6 were repeated until the volume of suspension in each vessel was one liter. At that point, the contents of both reactors were pumped into a two-liter reservoir adjacent to the hollow-fiber apparatus and were recirculated through the hollow-fiber device at 8 liters per minute. Permeate is continuously removed at 160 ml/min.
8. When the volume of the suspension was one liter, WFI was pumped into the reservoir at 160 ml/min. Permeate was continuously removed at 160 ml/min. The flow of WFI was stopped when seven liters had passed through the system.
9. The recirculation flow was reduced to 4 liters/minute and permeate flow was reduced to 80 ml/min. The pumping continues until the volume of the suspension is about 400 ml.
10. The pumps are stopped at this time and the suspension was drained from the hollow fiber apparatus into a sterile receiving vessel.
11. A concentrated solution of excipient was added to the product at this stage. The product was allowed to stir for 5 minutes.
12. The product was dispensed into vials for lyophilization, stoppering, and crimping.

Results: The microparticles were analyzed and the following results were obtained:
Encapsulation (determined by UV absorption): 4.0 µg DNA/mg particles
Supercoiling: 60% in microparticles
Microparticle diameter: 2.0 µm by number average, 6.2 µm by volume average; 80% were larger than 2.8 µm in diameter
Yield: 10.3 g (85.8%) in 650 ml water
PVA concentration: 1.1%

### EXAMPLE 6: 10X and up scale for microparticle production

The following describes the technique for production of microparticles at the 60-gram and higher scale.
1. An aqueous solution of DNA and sucrose, and a solution of PLGA and lipid in DCM. are simultaneously pumped through a continuous flow homogenizer to form the primary emulsion.
2. The primary emulsion and a solution of PVA/sucrose are simultaneously pumped through a second continuous flow homogenizer to form the secondary emulsion. The second emulsion is passed into a solvent removal device, forming a suspension of microparticles.
3. The suspension is pumped into a screen-type washer-dryer (e.g., a Sweco device), in which the product is washed with a first aqueous solution or WFI. This is followed by a washing with a second aqueous solution that contains an excipient (e.g., mannitol).
4. The product is dried inside the Sweco unit by a steady stream of dry, sterile air.
5. The dried product is removed from the Sweco unit through the discharge port into a sterile receiving vessel.
6. The dried product is filled into vials with a powder auger. This screw-like device raises the powder from the receiving vessel and dispenses it into injection vials that are then capped and crimped.

## Claims

1. A scalable continuous process for preparing nucleic acid-containing microparticles, the process comprising:
(a) providing a mixing chamber and a solvent removal device;
(b) continuously supplying a first emulsion to the mixing chamber, wherein the first emulsion comprises (i) an organic solution comprising a polymeric material and an organic solvent mixed with (ii) a first aqueous solution comprising a nucleic acid;
(c) continuously supplying a second aqueous solution to the mixing chamber, wherein the second aqueous solution comprises a surfactant;
(d) continuously emulsifying the first emulsion and the second aqueous solution in the mixing chamber to form a second emulsion, the second emulsion comprising nucleic acid, polymeric material, water, and organic solvent;
(e) continuously transferring the second emulsion from the mixing chamber to the solvent removal device; and
(f) removing the organic solvent from the second emulsion in the solvent removal device to form an aqueous suspension of nucleic acid-containing microparticles; wherein at least one of the first emulsion and the second aqueous solution further comprises a stabilizer.

2. The process of claim 1 wherein the first aqueous solution and the second aqueous solution are of essentially equal osmolarity.

3. The process of claim 2, wherein the stabilizer comprises a carbohydrate and a buffer.

4. The process of claim 3 wherein the stabilizer comprises sucrose and TRIS-EDTA.

5. The process of claim 4 wherein the stabilizer additionally comprises a lipid.

6. The process of claim 1 wherein the stabilizer comprises a lipid.

7. The process of claim 1, further comprising:
(g) providing a diafiltration apparatus;
(h) diluting the aqueous suspension with an aqueous wash solution:
(i) supplying the diluted aqueous suspension to the diafiltration apparatus; and
(j) removing an aqueous waste solution from the diluted aqueous suspension in the diafiltration apparatus, wherein the aqueous waste solution comprises at least some of the wash solution of step (h), to form in the diafiltration apparatus a purified aqueous suspension comprising nucleic acid-containing microparticles.

8. The process of claim 7, further comprising:
(k) concentrating the purified aqueous suspension in the diafiltration apparatus to form a concentrate; and
(l) transferring the concentrate into one or more vessels.

9. The process of claim 8 further comprising:
(m) lyophilizing, freeze-drying, or air-drying the concentrate in the one or more vessels, to form lyophilized, freeze-dried, or air-dried microparticles.

10. The process of claim 9 wherein the lyophilized or freeze-dried microparticles have a residual organic solvent level of less than 200 ppm.

11. The process of claim 10 wherein the lyophilized or freeze-dried microparticles have a residual organic solvent level of less than 50 ppm.

12. The process of claim 1, further comprising:
(g) contacting the aqueous suspension with a vibrating or non-vibrating fine-mesh screen;
(h) filtering the aqueous suspension through the screen to remove at least some of each of said first and second aqueous solutions and to retain the microparticles on the screen:
(i) washing the microparticles with at least one aqueous wash solution to produce washed microparticles; and
(j) drying the washed microparticles to produce dried microparticles.

13. The process of Claim 12, wherein the drying step comprises lyophilizing, freeze-drying, or air-drying the washed microparticles.

14. The process of claim 12, wherein the first aqueous wash solution is sterile water-for-injection at a temperature of about 2°C to about 8°C.

15. The process of claim 12, further comprising contacting the washed microparticles with an excipient, prior to the drying step.

16. The process of claim 12, further comprising:
(k) transferring the dried microparticles into one or more vessels.

17. The process of claim 1, wherein the mixing chamber comprises a homogenizer.

18. The process of claim 1, wherein the solvent removal device is a bioreactor.

19. The process of claim 1, wherein the second aqueous solution is supplied to the mixing chamber at a flow rate of between 0.1 and 20 l/min.

20. The process of claim 1, wherein the organic solvent is removed from the second emulsion in the solvent removal device by evaporation.

21. The process of claim 1, wherein the organic solvent is removed from the second emulsion by heating the second emulsion in the solvent removal device to between 30°C and 55°C.

22. The process of claim 1, wherein the organic solvent is removed from the second emulsion in the solvent removal device by an extraction process.

23. The process of claim 1, wherein the removal of the organic solvent from the second emulsion in the solvent removal device is facilitated by diluting the second emulsion in the solvent removal device.

24. The process of claim 1, wherein the organic solvent is removed from the second emulsion in the solvent removal device by applying a partial vacuum to the solvent removal device.

25. The process of claim 1, wherein the organic solvent comprises dichloromethane.

26. The process of claim 9, wherein each of the steps is carried out aseptically.

27. The process of claim 7, wherein the diafiltration apparatus comprises a hollow fiber system.

28. The process of claim 7, wherein steps (i) and (j) are carried out at a temperature of between about 2°C and about 8°C.

29. The process of claim 1, wherein at least about 50% of the nucleic acid in the microparticles is in the form of circular RNA molecules or supercoiled circular DNA molecules.

30. The process of claim 7, wherein at least about 50% of the nucleic acid in the microparticles in the purified aqueous suspension is in the form of circular RNA molecules or supercoiled circular DNA molecules.

31. The process of claim 9, wherein at least about 50% of the nucleic acid in the lyophilized or freeze-dried microparticles is in the form of supercoiled circular DNA molecules.

32. The process of claim 1, wherein the average diameter of microparticles is less than about 100 microns.

33. The process of claim 31, wherein the average diameter is less than about 20 microns.

34. The process of claim 32, wherein the average diameter is between about 0.5 and about 2.5 microns, inclusive.

35. The process of claim 1, wherein the polymeric material is a synthetic, biodegradable polymer.

36. The process of claim 35, wherein the polymer is poly-lactic-*co*-glycolic acid (PLGA).

37. The process of claim 36, wherein the ratio of lactic acid to glycolic acid in the PLGA is between about 1:2 and about 4:1 by weight.

38. The process of claim 37, wherein the ratio of lactic acid to glycolic acid in the PLGA is about 1:1 by weight.

39. The process of claim 36, wherein the PLGA has an average molecular weight in the range of 6,000 to 100,000.

40. The process of claim 1, wherein the second aqueous solution further comprises polyvinyl alcohol (PVA).

41. The process of claim 40, wherein the second aqueous solution further comprises a carbohydrate.

42. The process of claim 41, wherein the carbohydrate is sucrose.

43. The process of claim 1, wherein the emulsifying step (d) is carried out at between about 2°C and about 8°C.

44. The process of claim 1, wherein the average residence time of the first emulsion and the second aqueous solution in the mixing chamber is less than about 60 seconds.

45. The process of claim 44, wherein the average residence time of the first emulsion and the second aqueous solution in the mixing chamber is less than about 1 second.

46. The process of claim 1, wherein the average residence time of the second emulsion in the solvent removal device is less than about 3 hours.

47. The process of claim 1, further comprising:
(g) providing a diafiltration apparatus;
(h) diluting the aqueous suspension with an aqueous wash solution;
(i) supplying the diluted aqueous suspension to the diafiltration apparatus;
(j) removing an aqueous waste solution from the diluted aqueous suspension in the diafiltration apparatus, wherein the aqueous waste solution comprises at least some of the wash solution of step (h), to form in the diafiltration apparatus a purified aqueous suspension comprising nucleic acid-containing microparticles;
(k) washing the purified aqueous suspension to form a suspension of washed microparticles;
(l) concentrating the suspension of washed microparticles to form a concentrate;
(m) transferring the concentrate into one or more vessels; and
(n) lyophilizing, freeze-drying, or air-drying the concentrate in the one or more vessels, to form lyophilized, freeze-dried, or air-dried powder.

## Patentansprüche

1. Ein skalierbares kontinuierliches Verfahren zur Herstellung nukleinsäurehaltiger Mikropartikel, umfassend
a) Bereitstellen einer Mischkammer und einer Vorrichtung zur Lösungsmittelentfernung;
b) kontinuierliches Zuführen einer ersten Emulsion zur Mischkammer, wobei die erste Emulsion (i) eine organische, Polymer-enthaltende Lösung und ein organisches Lösungsmittel gemischt mit (ii) einer ersten, nukleinsäurehaltigen wässrigen Lösung enthält;
c) kontinuierliches Zuführen einer zweiten wässrigen Lösung zur Mischkammer, wobei die zweite wässrige Lösung ein Tensid enthält;
d) kontinuierliches Emulgieren der ersten Emulsion und der zweiten wässrigen Lösung in der Mischkammer zur Bildung einer zweiten Emulsion, wobei die zweite Emulsion Nukleinsäure, polymeres Material, Wasser und organisches Lösungsmittel enthält;
e) kontinuierliches Überführen der zweiten Emulsion von der Mischkammer zur Vorrichtung zur Lösungsmittelentfernung; und
f) Entfernen des organischen Lösungsmittels der zweiten Emulsion in der Vorrichtung zur Lösungsmittelentfernung zur Bildung einer wässrigen Suspension nukleinsäurehaltiger Mikropartikel; wobei mindestens eines von beiden, die erste Emulsion oder die zweite wässrige Lösung, ferner einen Stabilisator enthält.

2. Das Verfahren von Anspruch 1, wobei die erste wässrige Lösung und die zweite wässrige Lösung im wesentlichen die gleiche Osmolarität besitzen.

3. Das Verfahren von Anspruch 2, wobei der Stabilisator ein Kohlenhydrat und einen Puffer enthält.

4. Das Verfahren von Anspruch 3, wobei der Stabilisator Saccharose und TRIS-EDTA enthält.

5. Das Verfahren von Anspruch 4, wobei der Stabilisator zusätzlich ein Lipid enthält.

6. Das Verfahren von Anspruch 1, wobei der Stabilisator ein Lipid enthält.

7. Das Verfahren von Anspruch 1, ferner umfassend:
(g) Bereitstellen einer Diafiltrations-Apparatur;
(h) Verdünnen der wässrigen Suspension mit einer wässrigen Waschlösung;
(i) Zuführen der verdünnten, wässrigen Suspension zur Diafiltrations-Apparatur; und
(j) Abtrennen einer wässrigen Ausschußlösung von der verdünnten wässrigen Suspension in der Diafiltrations-Apparatur, wobei die wässrige Ausschußlösung zumindest etwas von der Waschlösung von Schritt (h) enthält, um in der Diafiltrations-Apparatur eine gereinigte wässrige Suspension zu bilden, die nukleinsäurehaltige Mikropartikel enthält.

8. Das Verfahren von Anspruch 1, ferner umfassend:
(k) Konzentrieren der gereinigten wässrigen Suspension in der Diafiltrations-Apparatur, um ein Konzentrat zu bilden; und
(l) Überführen des Konzentrats in ein oder mehrere Behältnisse.

9. Das Verfahren von Anspruch 8, ferner umfassend:
(m) Lyophilisieren, Gefriertrocknen, oder Lufttrocknen des Konzentrats in dem einen oder den mehreren Behältnissen, um lyophilisierte, gefriergetrocknete oder luftgetrocknete Mikropartikel zu bilden.

10. Das Verfahren von Anspruch 9, wobei die lyophilisierten oder gefriergetrockneten Mikropartikel weniger als 200 ppm an restlichem organischen Lösungsmittel enthalten.

11. Das Verfahren von Anspruch 10, wobei die lyophilisierten oder gefriergetrockneten Mikropartikel weniger als 50 ppm an restlichem organischen Lösungsmittel enthalten.

12. Das Verfahren von Anspruch 1, ferner umfassend:
(g) in Kontakt Bringen der wässrigen Suspension mit einem vibrierenden oder nicht-vibrierenden feinmaschigen Sieb,
(h) Filtrieren der wässrigen Suspension durch das Sieb, um zumindest etwas von jeweils der ersten und zweiten wässrigen Lösung zu entfernen und die Mikropartikel am Sieb zurückzuhalten;
(i) Waschen der Mikropartikel mit mindestens einer wässrigen Waschlösung, um gewaschene Mikropartikel herzustellen; und
(j) Trocknen der gewaschenen Mikropartikel, um getrocknete Mikropartikel herzustellen.

13. Das Verfahren von Anspruch 12, wobei der Trocknungsschritt das Lyophilisieren, Gefriertrocknen oder Lufttrocknen der gewaschenen Mikropartikel beinhaltet.

14. Das Verfahren von Anspruch 12, wobei die erste wässrige Waschlösung steriles "Wasser zur Injektion" mit einer Temperatur von ca. 2°C bis ca. 8°C ist.

15. Das Verfahren von Anspruch 12, das vor dem Trocknungsschritt ferner in Kontakt Bringen der gewaschenen Mikropartikel mit einem Hilfsstoff beinhaltet.

16. Das Verfahren von Anspruch 12, das ferner beinhaltet:
(k) Überführen der getrockneten Mikropartikel in ein oder mehrere Behältnisse.

17. Das Verfahren von Anspruch 1, wobei die Mischkammer einen Homogenisator enthält.

18. Das Verfahren von Anspruch 1, wobei die Vorrichtung zur Lösungsmittelentfernung ein Bioreaktor ist.

19. Das Verfahren von Anspruch 1, wobei die zweite wässrige Lösung der Mischkammer mit einer Flußrate von 0,1 bis 20 l/min zugeführt wird.

20. Das Verfahren von Anspruch 1, wobei das organische Lösungsmittel von der zweiten Emulsion in der Lösungsmittelentfernungsvorrichtung durch Verdampfen entfernt wird.

21. Das Verfahren von Anspruch 1, wobei das organische Lösungsmittel von der zweiten Emulsion durch Erhitzen der zweiten Emulsion auf 30°C bis 55°C in der Vorrichtung zur Lösungsmittelentfernung entfernt wird.

22. Das Verfahren von Anspruch 1, wobei das organische Lösungsmittel von der zweiten Emulsion in der Vorrichtung zur Lösungsmittelentfernung durch Extraktion entfernt wird.

23. Das Verfahren von Anspruch 1, wobei das Entfernen des organischen Lösungsmittels von der zweiten Emulsion in der Vorrichtung zur Lösungsmittelentfernung durch Verdünnen der zweiten Emulsion erleichtert wird.

24. Das Verfahren von Anspruch 1, wobei das organische Lösungsmittel von der zweiten Emulsion in der Vorrichtung zur Lösungsmittelentfernung durch Anlegen eines teilweisen Vakuums an die Vorrichtung zur Lösungsmittelentfernung entfernt wird.

25. Das Verfahren von Anspruch 1, wobei das organische Lösungsmittel Dichlormethan enthält.

26. Das Verfahren von Anspruch 9, wobei jeder Schritt unter aseptischen Bedingungen ausgeführt wird.

27. Das Verfahren von Anspruch 7, wobei die Diafiltrationsapparatur ein Hohlfasersystem enthält.

28. Das Verfahren von Anspruch 7, wobei die Schritte (i) und (j) bei einer Temperatur zwischen ca. 2°C und ca. 8°C ausgeführt werden.

29. Das Verfahren von Anspruch 1, wobei mindestens ca. 50% der Nukleinsäure in den Mikropartikeln in Form ringförmiger RNA-Moleküle oder in Supercoiled-Form vorliegender ringförmiger DNA-Moleküle vorliegen.

30. Das Verfahren von Anspruch 7, wobei mindestens ca. 50% der Nukleinsäure in den Mikropartikeln in der gereinigten wässrigen Suspension in Form ringförmiger RNA-Moleküle oder in Supercoiled-Form vorliegender ringförmiger DNA-Moleküle vorliegen.

31. Das Verfahren von Anspruch 9, wobei mindestens ca. 50% der Nukleinsäure in den lyophilisierten oder gefriergetrockneten Mikropartikeln in Form von in Supercoiled-Form vorliegender ringförmiger DNA-Moleküle vorliegen.

32. Das Verfahren von Anspruch 1, wobei der durchschnittliche Durchmesser der Mikropartikel weniger als ca. 100 Mikrometer beträgt.

33. Das Verfahren von Anspruch 31, wobei der durchschnittliche Durchmesser weniger als ca. 20 Mikrometer beträgt.

34. Das Verfahren von Anspruch 32, wobei der durchschnittliche Durchmesser zwischen ca. 0,5 und ca. 2,5 Mikrometer beträgt, inklusive.

35. Das Verfahren von Anspruch 1, wobei das polymere Material ein synthetisches, biologisch abbaubares Polymer ist.

36. Das Verfahren von Anspruch 35, wobei das Polymer ein Polymilchsäure-Polyglykolsäure-Copolymer (PLGA) ist.

37. Das Verfahren von Anspruch 36, wobei das Verhältnis von Milchsäure zu Glykolsäure im PLGA-Copolymer zwischen ca. 1:2 und ca. 4:1, bezogen auf das Gewicht, liegt.

38. Das Verfahren von Anspruch 37, wobei das Verhältnis von Milchsäure zu Glykolsäure im PLGA-Copolymer bei ca. 1:1, bezogen auf das Gewicht, liegt.

39. Das Verfahren von Anspruch 36, wobei das mittlere Molekulargewicht des PLGA-Copolymers im Bereich von 6.000 bis 100.000 liegt.

40. Das Verfahren von Anspruch 1, wobei die zweite wässrige Lösung ferner Polyvinylalkohol (PVA) enthält.

41. Das Verfahren von Anspruch 40, wobei die zweite wässrige Lösung ferner ein Kohlenhydrat enthält.

42. Das Verfahren von Anspruch 41, wobei das Kohlenhydrat Saccharose ist.

43. Das Verfahren von Anspruch 1, wobei der Emulgierungsschritt (d) zwischen ca. 2°C und ca. 8°C ausgeführt wird.

44. Das Verfahren von Anspruch 1, wobei die mittlere Verweilzeit der ersten Emulsion und der zweiten wässrigen Lösung in der Mischkammer weniger als ca. 60 Sekunden beträgt.

45. Das Verfahren von Anspruch 44, wobei die mittlere Verweilzeit der ersten Emulsion und der zweiten wässrigen Lösung in der Mischkammer weniger als ca. 1 Sekunde beträgt.

46. Das Verfahren von Anspruch 1, wobei die mittlere Verweilzeit der zweiten Emulsion in der Vorrichtung zur Lösungsmittelentfernung weniger als ca. 3 Stunden beträgt.

47. Das Verfahren von Anspruch 1, ferner umfassend:
(g) Bereitstellen einer Diafiltrations-Apparatur;
(h) Verdünnen der wässrigen Suspension mit einer wässrigen Waschlösung;
(i) Zuführen der verdünnten, wässrigen Suspension zur Diafiltrations-Apparatur;
(j) Abtrennen einer wässrigen Ausschußlösung von der verdünnten wässrigen Suspension in der Diafiltrations-Apparatur, wobei die wässrige Ausschußlösung zumindest etwas von der Waschlösung von Schritt (h) enthält, um in der Diafiltrations-Apparatur eine gereinigte wässrige Suspension zu bilden, die nukleinsäurehaltige Mikropartikel enthält;
(k) Waschen der gereinigten wässrigen Suspension, um eine Suspension von gewaschenen Mikropartikeln herzustellen;
(l) Konzentrieren der Suspension gewaschener Mikropatikel, um ein Konzentrat zu bilden;
(m) Überführen des Konzentrats in ein oder mehrere Behältnisse; und
(n) Lyophilisieren, Gefriertrocknen oder Lufttrocknen des Konzentrats in dem einen oder den mehreren Behältnissen, um ein lyophilisiertes, gefriergetrocknetes oder luftgetrocknetes Pulver zu bilden.

## Revendications

1. Procédé évolutif à débit continu pour produire des microparticules contenant des acides nucléiques, ce procédé comprenant les étapes consistant à:
(a) disposer d'une chambre de mélange et d'un dispositif d'élimination de solvant;
(b) alimenter en continu une première émulsion dans la chambre de mélange, cette première émulsion comprenant le mélange de
(i) une solution organique comprenant un matériau polymère et un solvant organique
(ii) une première solution aqueuse contenant un acide nucléique;
(c) alimenter en continu une deuxième solution aqueuse dans la chambre de mélange, cette deuxième solution aqueuse comprenant un agent tensio-actif;
(d) émulsionner en continu, dans la chambre de mélange, la première émulsion et la deuxième solution aqueuse afin de former une deuxième émulsion, cette deuxième émulsion comprenant un acide nucléique, un matériau polymère, de l'eau et un solvant organique;
(e) transférer en continu la deuxième émulsion de la chambre de mélange au dispositif d'élimination de solvant; et
(f) enlever, dans le dispositif d'élimination de solvant, le solvant organique de la deuxième émulsion, afin de former une suspension aqueuse de microparticules contenant de l'acide nucléique;
l'une au moins de la première émulsion et de la deuxième solution aqueuse comprenant en plus un agent stabilisant.

2. Procédé selon la revendication 1, dans lequel la première solution aqueuse et la deuxième solution aqueuse ont une osmolarité sensiblement égale.

3. Procédé selon la revendication 2, dans lequel l'agent stabilisant comprend un hydrate de carbone et un tampon.

4. Procédé selon la revendication 3, dans lequel l'agent stabilisant comprend du saccharose et du TRIS-EDTA.

5. Procédé selon la revendication 4, dans lequel l'agent stabilisant comprend en plus un lipide.

6. Procédé selon la revendication 1, dans lequel l'agent stabilisant comprend un lipide.

7. Procédé selon la revendication 1, comprenant en plus les étapes consistant à:
(g) disposer d'un appareil de diafiltration;
(h) diluer la suspension aqueuse avec une solution de lavage aqueuse;
(i) alimenter la suspension aqueuse diluée dans l'appareil de diafiltration; et
(j) éliminer de la suspension aqueuse diluée, dans l'appareil de diafiltration, une solution aqueuse formant déchets, cette solution aqueuse formant déchets comprenant au moins un peu de la solution de lavage de l'étape (h), afin de former dans l'appareil de diafiltration une suspension aqueuse purifiée comprenant des microparticules contenant de l'acide nucléique.

8. Procédé selon la revendication 7, comprenant en plus les étapes consistant à:
(k) concentrer dans l'appareil de diafiltration la suspension aqueuse purifiée afin de former un concentré; et
(l) transférer le concentré dans un ou plusieurs récipients.

9. Procédé selon la revendication 8, comprenant en plus les étapes consistant à:
(m) lyophiliser, cryodessécher ou sécher à l'air, dans lesdits un ou plusieurs récipients, le concentré afin de former des microparticules lyophilisées, cryodesséchées ou séchées à l'air.

10. Procédé selon la revendication 9, dans lequel les microparticules lyophilisées ou cryodesséchées ont une teneur en solvant organique résiduel inférieure à 200 ppm.

11. Procédé selon la revendication 10, dans lequel les microparticules lyophilisées ou cryodesséchées ont une teneur en solvant organique résiduel inférieure à 50 ppm.

12. Procédé selon la revendication 1, comprenant en plus les étapes consistant à:
(g) mettre en contact la suspension aqueuse avec un tamis à mailles fines, vibrant ou non vibrant;
(h) filtrer la suspension aqueuse sur le tamis afin d'éliminer au moins un peu de chacune desdites première et deuxième solutions aqueuses et retenir les microparticules sur le tamis;
(i) laver les microparticules avec au moins une solution de lavage aqueuse afin de produire des microparticules lavées; et
(j) sécher les microparticules lavées afin de produire des microparticules séchées.

13. Procédé selon la revendication 12, dans lequel l'étape de séchage comprend la lyophilisation, la cryodessiccation ou le séchage à l'air des microparticules lavées.

14. Procédé selon la revendication 12, dans lequel la première solution de lavage aqueuse est de l'eau stérile pour préparations injectables, se trouvant à une température d'environ 2°C à environ 8°C.

15. Procédé selon la revendication 12, comprenant en plus la mise en contact des microparticules lavées avec un excipient, avant l'étape de séchage.

16. Procédé selon la revendication 12, comprenant en plus l'étape consistant à:
(k) transférer les microparticules séchées dans un ou plusieurs récipients.

17. Procédé selon la revendication 1, dans lequel la chambre de mélange comprend un homogénéisateur.

18. Procédé selon la revendication 1, dans lequel le dispositif d'élimination de solvant est un bioréacteur.

19. Procédé selon la revendication 1, dans lequel la deuxième solution aqueuse est alimentée dans la chambre de mélange selon un débit dans la plage de 0,1 à 20 l/min.

20. Procédé selon la revendication 1, dans lequel le solvant organique est éliminé de la deuxième émulsion dans le dispositif d'élimination de solvant par évaporation.

21. Procédé selon la revendication 1, dans lequel le solvant organique est enlevé de la deuxième émulsion par chauffage de la deuxième émulsion dans le dispositif d'élimination de solvant à une température dans la plage de 30°C à 55°C.

22. Procédé selon la revendication 1, dans lequel le solvant organique est enlevé de la deuxième émulsion dans le dispositif d'élimination de solvant par un procédé d'extraction.

23. Procédé selon la revendication 1, dans lequel l'élimination du solvant organique de la deuxième émulsion dans le dispositif d'élimination de solvant est facilitée par la dilution de la deuxième émulsion dans le dispositif d'élimination de solvant.

24. Procédé selon la revendication 1, dans lequel le solvant organique est éliminé de la deuxième émulsion dans le dispositif d'élimination de solvant en appliquant un vide partiel dans le dispositif d'élimination de solvant.

25. Procédé selon la revendication 1, dans lequel le solvant organique comprend du dichlorométhane.

26. Procédé selon la revendication 9, dans lequel chacune des étapes est effectué en conditions aseptiques.

27. Procédé selon la revendication 7, dans lequel l'appareil de diafiltration comprend un système à fibres creuses.

28. Procédé selon la revendication 7, dans lequel les étapes (i) et (j) se font à une température dans la plage d'environ 2°C à environ 8°C.

29. Procédé selon la revendication 1, dans lequel au moins 50% environ de l'acide nucléique dans les microparticules se présentent sous la forme de molécules d'ARN circulaire ou de molécules d'ADN circulaire en superhélice.

30. Procédé selon la revendication 7, dans lequel au moins 50% environ des acides nucléiques dans les microparticules contenues dans la suspension aqueuse purifiée se présentent sous la forme de molécules d'ARN circulaire ou de molécules d'ADN circulaire en superhélice.

31. Procédé selon la revendication 9, dans lequel au moins 50% environ des acides nucléiques dans les microparticules lyophilisées ou cryodesséchées se présentent sous la forme de molécules d'ADN circulaire en superhélice.

32. Procédé selon la revendication 1, dans lequel le diamètre moyen des microparticules est inférieur à 100 microns environ.

33. Procédé selon la revendication 31, dans lequel le diamètre moyen est inférieur à 20 microns environ.

34. Procédé selon la revendication 32, dans lequel le diamètre moyen se situe dans la plage d'environ 0,5 micron à environ 2,5 microns, bornes comprises.

35. Procédé selon la revendication 1, dans lequel le matériau polymère est un polymère synthétique biodégradable.

36. Procédé selon la revendication 35, dans lequel le polymère est l'acide poly(lactique-co-glycolique) (PLGA).

37. Procédé selon la revendication 36, dans lequel le ratio acide lactique/acide glycolique dans le PLGA se situe dans la plage d'environ 1:2 à environ 4:1 en poids.

38. Procédé selon la revendication 37, dans lequel le ratio acide lactique/acide glycolique dans le PLGA est d'environ 1:1 en poids.

39. Procédé selon la revendication 36, dans lequel le PLGA présente une masse moléculaire moyenne dans la plage de 6 000 à 100 000.

40. Procédé selon la revendication 1, dans lequel la deuxième solution aqueuse comprend en plus de l'alcool polyvinylique (PVA).

41. Procédé selon la revendication 40, dans lequel la deuxième solution aqueuse comprend en plus un hydrate de carbone.

42. Procédé selon la revendication 41, dans lequel l'hydrate de carbone est le saccharose.

43. Procédé selon la revendication 1, dans lequel l'étape d'émulsification (d) est effectué dans la plage d'environ 2°C à environ 8°C.

44. Procédé selon la revendication 1, dans lequel le temps de séjour moyen de la première émulsion et de la deuxième solution aqueuse dans la chambre de mélange est inférieur à 60 secondes environ.

45. Procédé selon la revendication 44, dans lequel le temps de séjour moyen de la première émulsion et de la deuxième solution aqueuse dans la chambre de mélange est inférieur à 1 seconde environ.

46. Procédé selon la revendication 1, dans lequel le temps de séjour moyen de la deuxième émulsion dans le dispositif d'élimination de solvant est inférieur à 3 heures environ.

47. Procédé selon la revendication 1, comprenant en plus les étapes consistant à:
(g) disposer d'un appareil de diafiltration;
(h) diluer la suspension aqueuse avec une solution de lavage aqueuse;
(i) alimenter la suspension aqueuse diluée dans l'appareil de diafiltration;
(j) éliminer de la suspension aqueuse diluée, dans l'appareil de diafiltration, une solution aqueuse formant déchets dans laquelle la solution aqueuse formant déchets comprend au moins un peu de la solution de lavage de l'étape (h), afin de former dans l'appareil de diafiltration une suspension aqueuse purifiée comprenant des microparticules contenant de l'acide nucléique;
(k) laver la suspension aqueuse purifiée afin de former une suspension de microparticules lavées;
(l) concentrer la suspension de microparticules lavées afin de former un concentré;
(m) transférer le concentré dans un ou plusieurs récipients; et
(n) lyophiliser, cryodessécher ou sécher à l'air le concentré dans ledit un ou plusieurs récipients, afin de former une poudre lyophilisée, cryodesséchée ou séchée à l'air.
